(19) **Europäisches Patentamt / European Patent Office / Office européen des brevets**

(11) **EP 4 553 675 A1**

(12) **EUROPEAN PATENT APPLICATION**
published in accordance with Art. 153(4) EPC

(43) Date of publication:
14.05.2025 Bulletin 2025/20

(21) Application number: 23841768.7

(22) Date of filing: 15.03.2023

(51) International Patent Classification (IPC):
*G06F 16/53* (2019.01)   *G06T 7/00* (2017.01)

(52) Cooperative Patent Classification (CPC):
G06F 16/53; G06F 16/583; G06N 3/045;
G06N 3/0464; G06N 3/084; G06N 3/0985;
G06T 7/00; G06V 10/26; G06V 10/44; G06V 10/74;
G06V 10/80; G06V 10/82; G16H 30/20

(86) International application number:
PCT/CN2023/081658

(87) International publication number:
WO 2024/016691 (25.01.2024 Gazette 2024/04)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.07.2022 CN 202210868965

(71) Applicant: Huawei Cloud Computing
Technologies Co., Ltd.
Guiyang, Guizhou 550025 (CN)

(72) Inventors:
• PENG, Chengtao
  Guiyang, Guizhou 550025 (CN)
• ZHU, Senhua
  Guiyang, Guizhou 550025 (CN)
• TU, Dandan
  Guiyang, Guizhou 550025 (CN)

(74) Representative: Pfenning, Meinig & Partner mbB
Patent- und Rechtsanwälte
Theresienhöhe 11a
80339 München (DE)

(54) **IMAGE RETRIEVAL METHOD AND APPARATUS, MODEL TRAINING METHOD AND APPARATUS, AND STORAGE MEDIUM**

(57) This application relates to an image retrieval method, a model training method, an apparatus, and a storage medium. The image retrieval method may include: segmenting a target region in a to-be-retrieved image through a first neural network, to obtain at least one first feature, where the first feature is an intermediate feature extracted in a process of segmenting the target region; processing the to-be-retrieved image and the at least one first feature through a second neural network, to obtain a feature corresponding to the target region; generating, based on the feature corresponding to the target region, target code corresponding to the to-be-retrieved image; and retrieving a target image from a preset image set based on the target code. In this way, through information exchange between the first neural network and the second neural network, utilization of a feature extracted by the first neural network is improved, so that the second neural network generates accurate target code, thereby effectively improving accuracy of similarity retrieval.

Input a to-be-retrieved image into a target model, where the target model includes a first neural network and a second neural network — S401

Segment a target region in the to-be-retrieved image through the first neural network, to obtain at least one first feature, where the first feature is an intermediate feature extracted in a process of segmenting the target region — S402

Process the to-be-retrieved image and the at least one first feature through the second neural network, to obtain a feature corresponding to the target region; and generate, based on the feature corresponding to the target region, target code corresponding to the to-be-retrieved image — S403

Retrieve a target image from a preset image set based on the target code, where the preset image set includes a plurality of images and code corresponding to each of the plurality of images, and a similarity between code corresponding to the target image and the target code meets a preset condition — S404

FIG. 4

**Description**

This application claims priority to Chinese Patent Application No. 202210868965.7, filed with the China National Intellectual Property Administration on July 22, 2022 and entitled "IMAGE RETRIEVAL METHOD, MODEL TRAINING METHOD, APPARATUS, AND STORAGE MEDIUM", which is incorporated herein by reference in its entirety.

**TECHNICAL FIELD**

This application relates to the field of image processing technologies, and in particular, to an image retrieval method, a model training method, an apparatus, and a storage medium.

**BACKGROUND**

With development of medical imaging technologies, medical imaging (for example, lung electronic computed tomography (Computed Tomography, CT)) has become a mainstream detection method for some diseases (for example, pneumonia). Due to factors such as a large amount of clinical data, a low resolution of a medical image, and an unnoticeable lesion feature, errors are inevitable in complete manual screening of diseases and complete manual designation of related diagnosis and treatment plans. In addition, clinical experience accumulation of a doctor and memory of disease features play an important role in diagnosis and treatment of related diseases. However, this manner has strong subjectivity and uncertainty. Finally, for complex and severe diseases or diseases that are not common in clinic, a doctor usually needs to query whether there is a related case history in an entire medical community, and then makes a further judgment on the disease. However, a huge medical database makes it difficult for a clinician to find a related similar case or find a most matched similar case, which is not conducive to subsequent diagnosis and treatment on the disease. Therefore, an automated similar cases retrieval technology is particularly important in clinical application.

Due to a powerful information representation capability of deep learning (Deep Learning, DL) (for example, a convolutional neural network (Convolutional Neural Network, CNN)), an existing clinical similar cases retrieval method is implemented mainly depending on deep learning. However, accuracy of the retrieval still needs to be improved.

**SUMMARY**

In view of this, an image retrieval method, a model training method, an apparatus, an electronic device, and a storage medium are provided.

According to a first aspect, an embodiment of this application provides an image retrieval method. The method includes: inputting a to-be-retrieved image into a target model, where the target model includes a first neural network and a second neural network; segmenting a target region in the to-be-retrieved image through the first neural network, to obtain at least one first feature, where the first feature is an intermediate feature extracted in a process of segmenting the target region; processing the to-be-retrieved image and the at least one first feature through the second neural network, to obtain a feature corresponding to the target region; generating, based on the feature corresponding to the target region, target code corresponding to the to-be-retrieved image; and retrieving a target image from a preset image set based on the target code, where the preset image set includes a plurality of images and code corresponding to each of the plurality of images, and a similarity between code corresponding to the target image and the target code meets a preset condition.

Based on the technical solution, the target region in the to-be-retrieved image is segmented through the first neural network, and the intermediate feature (namely, the first feature) extracted in the process of segmenting the target region is transferred to the second neural network, so that the feature extracted by the first neural network is shared with the second neural network. Because the first feature includes information about the target region, in a process of processing the to-be-retrieved image, the second neural network may focus on the target region with reference to the first feature, to eliminate interference from invalid information or interference information of a non-target region. The obtained feature corresponding to the target region can better represent the target region. The accurate target code is generated based on the feature corresponding to the target region, and the accurate target code is used to retrieve the target image. In this way, through information exchange between the first neural network and the second neural network, utilization of a feature extracted by the first neural network is improved, so that the second neural network generates accurate target code, thereby effectively improving accuracy of similarity retrieval. In addition, the target model can complete both segmentation of the target region and retrieval of the target image, so that two functions are implemented by using one model. This is more economical and practical.

According to the first aspect, in a first possible implementation of the first aspect, the processing the to-be-retrieved image and the at least one first feature through the second neural network, to obtain a feature corresponding to the target region includes: obtaining a first feature that is in the at least one first feature and that corresponds to at least one convolutional layer in the second neural network, where a size of the first feature corresponding to the at least one convolutional layer is the same as a size of a feature extracted by the at least one convolutional layer; fusing the feature extracted by the at least one convolutional layer and the first feature corresponding to the at least one convolutional layer, to obtain a feature corresponding to the at least one convolutional layer; and processing the feature corresponding to the at least one convolutional layer, to obtain the feature corresponding to the target region.

Based on the technical solution, features of a same size may include same or similar information; a first feature that has a same size as a feature extracted by a convolutional layer in the second neural network is transferred to the convolutional layer; and the intermediate feature extracted by the convolutional layer is fused with the first feature, and a feature obtained through fusion may be used as a feature corresponding to the convolutional layer. Therefore, feature extraction performance of the convolutional layer on the target region in the to-be-retrieved image is enhanced, and the finally obtained feature corresponding to the target region can better represent the target region.

According to the first aspect or the first possible implementation of the first aspect, in a second possible implementation of the first aspect, the method further includes: displaying the to-be-retrieved image; and determining, in response to a mark operation performed by a user on the to-be-retrieved image, a target seed point corresponding to the to-be-retrieved image; and inputting the target seed point into the target model; and the segmenting a target region in the to-be-retrieved image through the first neural network, to obtain at least one first feature includes: performing region growing based on the target seed point, to obtain an initial region; and segmenting the initial region and the target region in the to-be-retrieved image through the first neural network, to obtain the at least one first feature.

Based on the technical solution, the target model obtains the to-be-retrieved image and the target seed point selected by the user, may perform region growing based on the target seed point selected by the user, and in the process of segmenting the target region in the to-be-retrieved image through the first neural network, uses the initial region obtained through region growing as a reference. This further improves accuracy of an obtained segmentation result of the target region. Correspondingly, the intermediate feature extracted in the process of segmenting the target region through the first neural network may include more accurate information about the target region, and the intermediate feature is transferred to the second neural network, so that the second neural network generates more accurate target code. This further improves the accuracy of similarity retrieval.

According to the first aspect or the foregoing possible implementations of the first aspect, in a third possible implementation of the first aspect, the method further includes: displaying at least one candidate category; determining a target category in response to a selection operation performed by the user in the at least one candidate category, where the target category indicates a category corresponding to the target region; and determining the target model from at least one preset model based on the target category.

Based on the technical solution, through interaction, the user is allowed to select, from candidate categories, a category (or the target category) on which similarity retrieval is expected to be performed. This improves an interaction capability of the user, so that the user can dynamically adjust the target category based on a requirement, and implement similarity retrieval through a corresponding target model. Therefore, a problem in a related technology that similarity retrieval can be performed only on a single fixed target category is resolved, and practicability is higher.

According to the first aspect or the foregoing possible implementations of the first aspect, in a fourth possible implementation of the first aspect, the preset image set further includes: a region segmentation result corresponding to each of the plurality of images; and the method further includes: displaying a segmentation result obtained by segmenting the target region and a region segmentation result corresponding to the target image.

Based on the technical solution, the segmentation result obtained by segmenting the target region in the to-be-retrieved image through the first neural network and the region segmentation result corresponding to the retrieved target image are displayed, to facilitate viewing and comparison by the user.

According to a second aspect, an embodiment of this application provides a model training method. The method includes: inputting a sample image group into a target preset model, where the sample image group includes three sample images, categories of two sample images are the same, a category of a $3^{rd}$ sample image is different from the categories of the two sample images, and the target preset model includes a first preset neural network and a second preset neural network; performing region segmentation on each sample image in the sample image group through the first preset neural network, to obtain a first segmentation result corresponding to each sample image; processing, through the second preset neural network, each sample image and at least one first feature corresponding to each sample image, to obtain a feature corresponding to a segmented region in each sample image; generating, based on the feature corresponding to the segmented region in each sample image, code corresponding to each sample image, where the first feature is an intermediate feature extracted in a process of performing the region segmentation; and training the target preset model based on the code corresponding to each sample image and the first segmentation result corresponding to each sample image, to obtain a target model.

Based on the technical solution, region segmentation is performed on each sample image in the sample image group through the first preset neural network, and the intermediate feature (namely, the first feature corresponding to each sample image) extracted in the process of performing region segmentation is transferred to the second preset neural network, so that the feature extracted by the first preset neural network is shared with the second preset neural network.

Because the first feature corresponding to each sample image includes information about the segmented region, in a process of processing each sample image, the second preset neural network may focus on the segmented region with reference to the first feature corresponding to each sample image, to eliminate interference from irrelevant information. The obtained feature corresponding to the segmented region can better represent the segmented region. The code corresponding to each sample image is generated based on the feature corresponding to the segmented region. In this way, in a training process, through information exchange between the first preset neural network and the second preset neural network, utilization of a feature extracted by the first preset neural network is improved, and the code that corresponds to each sample image and that is generated by the second preset neural network through continuous learning can be used to distinguish between sample images of different categories more effectively. Therefore, the target model with excellent similarity retrieval performance is obtained. In addition, in a related technology, in a similarity retrieval manner, a plurality of networks are usually trained in phases, and a training process is complex. In this technical solution, in a training process, information exchange between the first preset neural network and the second preset neural network is added, and the feature extracted by the first preset neural network is transferred to the second preset neural network, so that the first preset neural network and the second preset neural network are no longer independent of each other and are integrated into an entire network (namely, the target preset model), and network training can be performed in an end-to-end manner, to reduce complexity of network training and improve training efficiency.

According to the second aspect, in a first possible implementation of the second aspect, the processing, through the second preset neural network, each sample image and at least one first feature corresponding to each sample image, to obtain a feature corresponding to a segmented region in each sample image includes: obtaining a first feature that is in at least one first feature corresponding to a first sample image and that corresponds to at least one convolutional layer in the second preset neural network, where the first sample image is any image in the sample image group, and a size of the first feature corresponding to the at least one convolutional layer is the same as a size of a feature extracted by the at least one convolutional layer; fusing the feature extracted by the at least one convolutional layer and the first feature corresponding to the at least one convolutional layer, to obtain a feature corresponding to the at least one convolutional layer; and processing the feature corresponding to the at least one convolutional layer, to obtain a feature corresponding to a segmented region in the first sample image.

Based on the technical solution, features of a same size may include same or similar information; a first feature that corresponds to the first sample image and that has a same size as a feature extracted by a convolutional layer in the preset second neural network is transferred to the convolutional layer; and the feature extracted by the convolutional layer is fused with the first feature, and a feature obtained through fusion may be used as a feature corresponding to the convolutional layer. Therefore, feature extraction performance of the convolutional layer on the segmented region in the first sample image is enhanced, and the finally obtained feature corresponding to the segmented region can better represent the segment region in the first sample image.

According to the second aspect or the first possible implementation of the second aspect, in a second possible implementation of the second aspect, the training the target preset model based on the code corresponding to each sample image and the first segmentation result corresponding to each sample image, to obtain a target model includes: determining a first loss function value based on the first segmentation result corresponding to each sample image and a label corresponding to each sample image; determining a second loss function value based on the code corresponding to each sample image; determining a target loss function value based on the first loss function value and the second loss function value; and updating the target preset model based on the target loss function value until a preset training end condition is met, to obtain the target model.

**In** some examples, a first loss function may be a dice loss function, and a second loss function may be a triplet loss function.

According to the second aspect or the foregoing possible implementations of the second aspect, in a third possible implementation of the second aspect, the method further includes: displaying each sample image; determining, in response to a mark operation performed by a user on each sample image, a seed point corresponding to each sample image; and inputting the seed point corresponding to each sample image into the target preset model; and the performing region segmentation on each sample image in the sample image group through the first preset neural network, to obtain a first segmentation result corresponding to each sample image includes: performing region growing based on a seed point corresponding to a first sample image, to obtain a second segmentation result corresponding to the first sample image; and processing, through the first preset neural network, the first sample image and the second segmentation result corresponding to the first sample image, to obtain a first segmentation result corresponding to the first sample image.

Based on the technical solution, region growing is performed based on the seed point selected by the user in each sample image, and in the process of performing region segmentation on each sample image through the first preset neural network, a second segmentation result obtained through region growing is used as a reference. This further improves accuracy of an obtained first segmentation result. Correspondingly, the intermediate feature extracted in the process of performing region segmentation through the first preset neural network may include more accurate information about the segmented region, and the intermediate feature is transferred to the second preset neural network, so that the code generated by the second preset neural network can be used to distinguish between sample images of different categories more effectively. Therefore, performance of the target model obtained through training is further improved.

According to the third possible implementation of the second aspect, in a fourth possible implementation of the second aspect, the method further includes: determining a difference between the first segmentation result corresponding to the first sample image and the second segmentation result corresponding to the first sample image; and determining, based on the difference, a label corresponding to the first sample image; and when the difference is greater than a preset value, using the first segmentation result corresponding to the first sample image as the label corresponding to the first sample image, or when the difference is not greater than a preset value, using the second segmentation result corresponding to the first sample image as the label corresponding to the first sample image.

Based on the technical solution, the second segmentation result obtained by performing region growing based on the seed point or the first segmentation result outputted by the first preset neural network is used as the label corresponding to each sample image to train the first preset neural network, and a large quantity of manually marked labels do not need to be obtained, thereby improving practicability and feasibility of the training method.

According to a third aspect, an embodiment of this application provides an image retrieval apparatus. The apparatus includes: an input module, configured to input a to-be-retrieved image into a target model, where the target model includes a first neural network and a second neural network; a segmentation module, configured to segment a target region in the to-be-retrieved image through the first neural network, to obtain at least one first feature, where the first feature is an intermediate feature extracted in a process of segmenting the target region; an encoding module, configured to process the to-be-retrieved image and the at least one first feature through the second neural network, to obtain a feature corresponding to the target region; and generate, based on the feature corresponding to the target region, target code corresponding to the to-be-retrieved image; and a retrieval module, configured to retrieve a target image from a preset image set based on the target code, where the preset image set includes a plurality of images and code corresponding to each of the plurality of images, and a similarity between code corresponding to the target image and the target code meets a preset condition.

According to the third aspect, in a first possible implementation of the third aspect, the encoding module is further configured to: obtain a first feature that is in the at least one first feature and that corresponds to at least one convolutional layer in the second neural network, where a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain the feature corresponding to the target region.

According to the third aspect or the first possible implementation of the third aspect, in a second possible implementation of the third aspect, the apparatus further includes: a display module, configured to display the to-be-retrieved image; and a marking module, configured to determine, in response to a mark operation performed by a user on the to-be-retrieved image, a target seed point corresponding to the to-be-retrieved image, where the input module is further configured to input the target seed point into the target model; and the segmentation module is further configured to: perform region growing based on the target seed point, to obtain an initial region; and process the initial region and the to-be-retrieved image through the first neural network, to obtain the at least one first feature.

According to the third aspect or the foregoing possible implementation of the third aspect, in a third possible implementation of the third aspect, the apparatus further includes: a display module, configured to display at least one candidate category; a selection module, configured to determine a target category in response to a selection operation performed by the user in the at least one candidate category, where the target category indicates a category corresponding to the target region; and a determining module, configured to determine the target model from at least one preset model based on the target category.

According to the third aspect or the foregoing possible implementations of the third aspect, in a fourth possible implementation of the third aspect, the preset image set further includes: a region segmentation result corresponding to each of the plurality of images; and the apparatus further includes: a display module, configured to display a segmentation result obtained by segmenting the target region and a region segmentation result corresponding to the target image.

According to a fourth aspect, an embodiment of this application provides a model training apparatus. The apparatus includes: an input module, configured to input a sample image group into a target preset model, where the sample image group includes three sample images, categories of two sample images are the same, a category of a $3^{rd}$ sample image is different from the categories of the two sample images, and the target preset model includes a first preset neural network and a second preset neural network; a segmentation module, configured to perform region segmentation on each sample image in the sample image group through the first preset neural network, to obtain a first segmentation result corresponding to each sample image; a feature module, configured to process, through the second preset neural network, each sample image and at least one first feature corresponding to each sample image, to obtain a feature corresponding to a segmented region in each sample image; and generate, based on the feature corresponding to the segmented region in each sample image, code corresponding to each sample image, where the first feature is an intermediate feature extracted in a process of performing the region segmentation; and a training module, configured to train the target preset model based on the code corresponding to each sample image and the first segmentation result corresponding to each sample image, to obtain a target model.

According to the fourth aspect, in a first possible implementation of the fourth aspect, the feature module is further configured to: obtain a first feature that is in at least one first feature corresponding to a first sample image and that corresponds to at least one convolutional layer in the second preset neural network, where the first sample image is any image in the sample image group, and a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain a feature corresponding to a segmented region in the first sample image.

According to the fourth aspect or the first possible implementation of the fourth aspect, in a second possible implementation of the fourth aspect, the training module is further configured to: determine a first loss function value based on the first segmentation result corresponding to each sample image and a label corresponding to each sample image; determine a second loss function value based on the code corresponding to each sample image; determine a target loss function value based on the first loss function value and the second loss function value; and update the target preset model based on the target loss function value until a preset training end condition is met, to obtain the target model.

According to the fourth aspect or the foregoing possible implementations of the fourth aspect, in a third possible implementation of the fourth aspect, the apparatus further includes: a display module, configured to display each sample image; and a marking module, configured to determine, in response to a mark operation performed by a user on each sample image, a seed point corresponding to each sample image, where the input module is further configured to input the seed point corresponding to each sample image into the target preset model; and the segmentation module is further configured to: perform region growing based on a seed point corresponding to a first sample image, to obtain a second segmentation result corresponding to the first sample image; and process, through the first preset neural network, the first sample image and the second segmentation result corresponding to the first sample image, to obtain a first segmentation result corresponding to the first sample image.

According to the fourth aspect or the third possible implementation of the fourth aspect, in a fourth possible implementation of the fourth aspect, the apparatus further includes: a difference determining module, configured to determine a difference between the first segmentation result corresponding to the first sample image and the second segmentation result corresponding to the first sample image; and a label determining module, configured to: determine, based on the difference, a label corresponding to the first sample image; and when the difference is greater than a preset value, use the first segmentation result corresponding to the first sample image as the label corresponding to the first sample image, or when the difference is not greater than a preset value, use the second segmentation result corresponding to the first sample image as the label corresponding to the first sample image.

According to a fifth aspect, an embodiment of this application provides an electronic device, including: a processor; and a memory configured to store instructions executable by the processor. The processor is configured to implement the image retrieval method according to one or more of the first aspect or the foregoing possible implementations of the first aspect or implement the model training method according to one or more of the second aspect or the foregoing possible implementations of the second aspect when executing the instructions. In some examples, the electronic device may be provided with a display apparatus.

According to a sixth aspect, an embodiment of this application provides a computer-readable storage medium, storing computer program instructions. When the computer program instructions are executed by a processor, the image retrieval method according to one or more of the first aspect or the foregoing possible implementations of the first aspect is implemented, or the model training method according to one or more of the second aspect or the foregoing possible implementations of the second aspect is implemented.

According to a seventh aspect, an embodiment of this application provides a computer program product. When the computer program product runs on a computer, the computer is enabled to perform the image retrieval method according to one or more of the first aspect or the foregoing possible implementations of the first aspect, or perform the model training method according to one or more of the second aspect or the foregoing possible implementations of the second aspect.

For technical effects of the third aspect to the seventh aspect, refer to the first aspect or the second aspect.

**BRIEF DESCRIPTION OF DRAWINGS**

Accompanying drawings included in the specification and constructing a part of the specification jointly show example embodiments, features, and aspects of this application, and are intended to explain the principles of this application.

FIG. 1 is a diagram of an overall feature-based similar cases retrieval manner according to an embodiment of this application;

FIG. 2 is a diagram of a local feature-based similar cases retrieval manner according to an embodiment of this application;

FIG. 3 is a diagram of an applicable scenario of an image retrieval method according to an embodiment of this application;

FIG. 4 is a flowchart of an image retrieval method according to an embodiment of this application;

FIG. 5 is a diagram of transferring a feature between a first neural network and a second neural network according to an embodiment of this application;

FIG. 6 is a diagram of an image retrieval method according to an embodiment of this application;

FIG. 7 is a flowchart of an image retrieval method according to an embodiment of this application;

FIG. 8 is a diagram of an image retrieval method according to an embodiment of this application;

FIG. 9 is a flowchart of an image retrieval method according to an embodiment of this application;

FIG. 10 is a flowchart of a model training method according to an embodiment of this application;

FIG. 11 is a diagram of calculating a triplet loss function value according to an embodiment of this application;

FIG. 12 is a flowchart of a model training method according to an embodiment of this application;

FIG. 13 is a diagram of determining a label corresponding to each sample image according to an embodiment of this application;

FIG. 14 is a diagram of a model training method according to an embodiment of this application;

FIG. 15 is a diagram of a structure of an image retrieval apparatus according to an embodiment of this application;

FIG. 16 is a diagram of a structure of a model training apparatus according to an embodiment of this application; and

FIG. 17 is a diagram of a structure of an electronic device according to an embodiment of this application.

## DESCRIPTION OF EMBODIMENTS

The following will describe various example embodiments, features, and aspects of this application in detail with reference to the accompanying drawings. Identical accompanying symbols in the accompanying drawings represent elements that have same or similar functions. Although various aspects of embodiments are illustrated in the accompanying drawing, the accompanying drawings are not necessarily drawn in proportion unless otherwise specified.

Reference to "an embodiment" or "some embodiments" described in this specification means that one or more embodiments of this application include a specific feature, structure, or characteristic described with reference to embodiment. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

In this application, "at least one" means one or more, and "a plurality of" means two or more. "And/or" describes an association relationship between associated objects and indicates that three relationships may exist. For example, A and/or B may indicate the following three cases: Only A exists, both A and B exist, and only B exists, where A and B may be singular or plural. The character "/" usually indicates an "or" relationship between the associated objects. "At least one of the following items (pieces)" or a similar expression thereof refers to any combination of these items, including any combination of singular items (pieces) or plural items (pieces). For example, at least one of a, b, or c may indicate: a, b, c, a-b, a-c, b-c, or a-b-c, where a, b, and c may be singular or plural.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

The specific term "example" herein means "used as an example, embodiment or illustrative". Any embodiment described as "example" is not necessarily explained as being superior or better than other embodiments. In addition, for better illustration of this application, various specific details are given in the following specific implementations. A person of ordinary skill in the art should understand that this application may also be implemented without the specific details.

To better describe solutions in embodiments of this application, the following first describes related terms and concepts that may be used in embodiments of this application.

### 1. Deep learning

The deep learning is a machine learning technology based on a deep neural network algorithm. A main feature of the deep learning is to process and analyze data by using a plurality of processing layers formed through multiple nonlinear transformations. The deep learning is mainly applied to scenarios such as perception and decision-making in the field of artificial intelligence, such as image and speech recognition, natural language translation, and computer games.

### 2. Convolutional neural network

The convolutional neural network is a deep neural network of a convolutional structure. The convolutional neural network includes a feature extractor including a convolutional layer and a sub-sampling layer. The feature extractor may be considered as a filter. A convolution process may be considered as performing convolution by using the same trainable filter and an input image or a convolutional feature map (feature map). The convolutional layer is a neuron layer that is in the convolutional neural network and at which convolution processing is performed on an input signal. At the convolutional layer of the convolutional neural network, one neuron may be connected only to some adjacent-layer neurons. One convolutional layer usually includes several feature planes, and each feature plane may include some neurons that are in a rectangular arrangement. Neurons on a same feature plane share a weight, and the weight shared herein is a convolution kernel. Weight sharing may be understood as that an image information extraction manner is irrelevant to a location. A principle implied herein is that statistical information of a part of an image is the same as that of other parts. This means that image information learned in a part can also be used in another part. Therefore, the same image information obtained through learning can be used for all locations on the image. At a same convolutional layer, a plurality of convolution kernels may be used to extract different image information. Usually, a larger quantity of convolution kernels indicates richer image information reflected in a convolution operation. The convolution kernel may be initialized in a form of a random-size matrix. In a training process of the convolutional neural network, the convolution kernel may obtain an appropriate weight through learning. In addition, benefits directly brought by weight sharing are that connections between layers of the convolutional neural network are reduced, and an overfitting risk is reduced.

### 3. Loss function

In a process of training a deep neural network, because it is expected that an output of the deep neural network is as much as possible close to a predicted value that is actually expected, a predicted value of a current network and a target value that is actually expected may be compared, and then a weight vector of each layer of the neural network is updated based on a difference between the predicted value and the target value (certainly, there is usually an initialization process before the first update, to be specific, parameters are preconfigured for all layers of the deep neural network). For example, if the predicted value of the network is large, the weight vector is adjusted to decrease the predicted value, and adjustment is continuously performed, until the neural network can predict the target value that is actually expected. Therefore, "how to obtain, through comparison, the difference between the predicted value and the target value" needs to be predefined. This is a loss function (loss function) or an objective function (objective function). The loss function and the objective function are important equations used to measure the difference between the predicted value and the target value. The loss function is used as an example. A higher output value (loss) of the loss function indicates a larger difference. Therefore, training of the deep neural network is a process of minimizing the loss as much as possible.

### 4. Contrastive learning (Contrastive Learning, CL)

The contrastive learning is a learning manner of learning a unique feature of a specific type by comparing a difference between images of different types.

### 5. Pseudo label (Pseudo Label, PL)

The pseudo label is a rough contour of a segmented target generated by using a series of technical means. The contour cannot accurately represent contour information of the target. In a network training process, the pseudo label can only be used as an auxiliary label, and cannot be used as a training label with a full confidence.

### 6. Region growing (Region Growing, GR)

The region growing refers to a process of clustering surrounding pixels with similar features by using a point (namely, a seed point) that is set during initialization, and finally outputting a region with a same feature.

7. Similarity retrieval (Similarity Search, SS)

The similarity retrieval is also referred to as similarity search, and refers to retrieving an image whose content is highly similar to content of a current image by using a series of technical means.

8. Lesion segmentation (Lesion Segmentation)

The lesion segmentation refers to extracting lesion information based on collected imaging information through deep learning and other methods to predict a region in which a lesion is located.

9. Similar cases retrieval (Similar cases recommendation)

The similar cases retrieval is also referred to as similar cases recommendation, and is a technical means that provides a diagnosis basis for a doctor to confirm a suspected case or provide a similar medical treatment solution for a confirmed patient based on related image information of a previous confirmed case.
In a related technology, there are mainly two deep learning-based similar cases retrieval manners.

(1) Overall feature-based similar cases retrieval manner

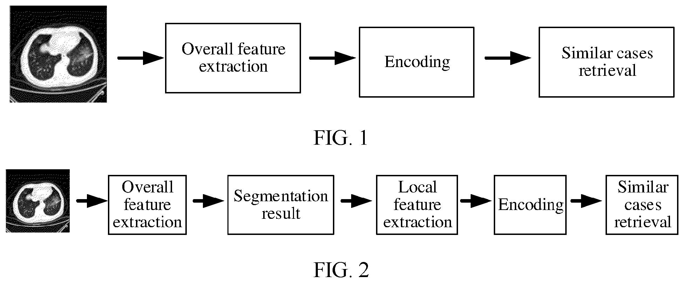

FIG. 1 is a diagram of an overall feature-based similar cases retrieval manner according to an embodiment of this application. As shown in FIG. 1, a two-dimensional medical image is inputted into a convolutional neural network, an overall feature of the image is extracted and encoded, and similar cases retrieval is performed based on code. In this manner, overall information of the medical image is directly extracted for similar cases retrieval. In addition to extracting information in a lesion region in the image, invalid information or even interference information outside the lesion region is further included. Affected by the information, accuracy of similar cases retrieval is low.

(2) Local feature-based similar cases retrieval manner

FIG. 2 is a diagram of a local feature-based similar cases retrieval manner according to an embodiment of this application. As shown in FIG. 2, a two-dimensional medical image is inputted into a convolutional neural network, an overall feature of the image is extracted, and a lesion region is segmented; and then a local feature of a lesion region segmentation result is extracted and encoded by another convolutional neural network, and similar cases retrieval is performed based on code. This manner is performed in two phases: a segmentation phase and a similar cases retrieval phase. To be specific, the lesion region is first segmented, and then the lesion region segmentation result is directly used for similar cases retrieval. Only result transfer is directly performed between the two phases. There is no effective information exchange between the segmentation phase and the similar cases retrieval phase. As a result, information utilization is low, and accuracy of similar cases retrieval is low.
To resolve the foregoing problem in the related technology, an embodiment of this application provides an image retrieval method.

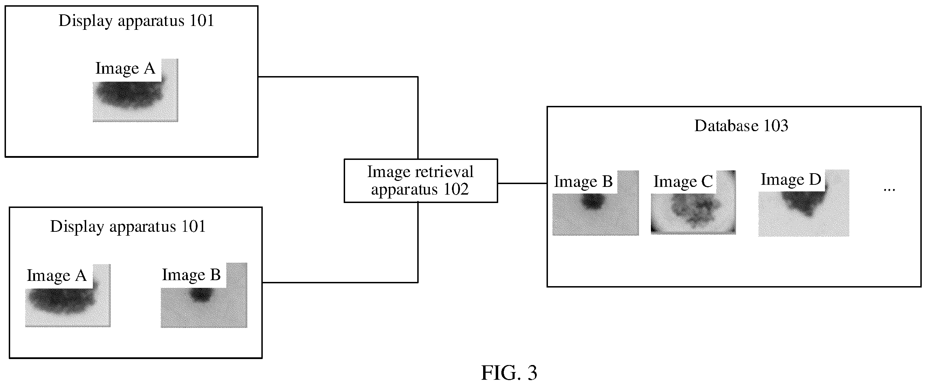

FIG. 3 is a diagram of an applicable scenario of an image retrieval method according to an embodiment of this application. As shown in FIG. 3, when a doctor views an image A of a patient through a display apparatus 101, the image retrieval method provided in this embodiment of this application is performed through an image retrieval apparatus 102 (for detailed descriptions, refer to the following). An image having a lesion similar to a lesion in the image A is retrieved from a plurality of existing images (only an image B, an image C, and an image D are shown in the figure) in a database 103, and the image may be presented to the doctor through the display apparatus 101 (as shown in the figure, the image B having a lesion similar to the lesion in the image A is retrieved, and the image A and the image B are displayed on the display apparatus 101), used as a comparison reference and assisting the doctor in medical diagnosis.
A type of the image retrieval apparatus is not limited in this embodiment of this application.
For example, the image retrieval apparatus may be independently disposed, may be integrated into another apparatus, or may be implemented by using software or a combination of software and hardware.
For example, the image retrieval apparatus may alternatively be a device or a system having a data processing capability, or a component or a chip disposed in the device or the system. For example, the image retrieval apparatus may be a cloud server, a desktop computer, a portable computer, a network server, a palmtop computer (personal digital assistant, PDA), a mobile phone, a tablet computer, a wireless terminal device, an embedded device, a medical device, another device with a data processing function, or a component or a chip in the device.
For example, the image retrieval apparatus may alternatively be a chip or a processor having a processing function, and the image retrieval apparatus may include a plurality of processors. The processor may be a single-core (single-CPU) processor, or may be a multi-core (multi-CPU) processor.

It should be noted that the application scenario described in this embodiment of this application is intended to describe the technical solutions in embodiments of this application more clearly, and does not constitute a limitation on the technical solutions provided in embodiments of this application. A person of ordinary skill in the art may know that: With emergence of other similar or new scenarios, the technical solutions provided in embodiments of this application are also applicable to similar technical problems, for example, may be applied to scenarios such as similar product retrieval.

The following describes the technical solutions provided in this application from two aspects: an application side and a training side. First, the image retrieval method provided in this embodiment of this application is described in detail from the application side.

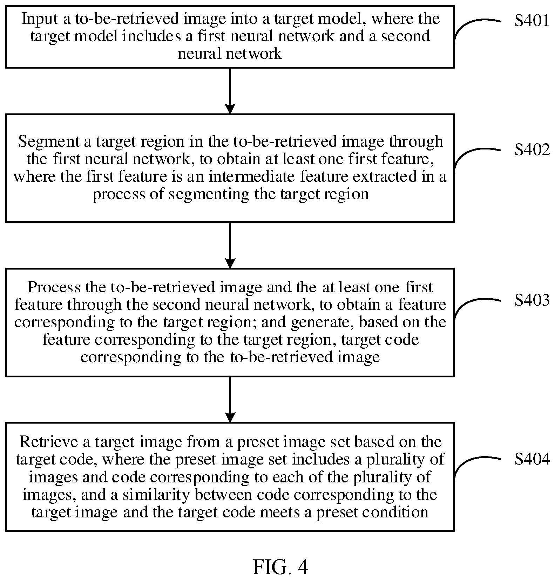

FIG. 4 is a flowchart of an image retrieval method according to an embodiment of this application. The method may be performed by the foregoing image retrieval apparatus. As shown in FIG. 4, the method may include the following steps.

S401: Input a to-be-retrieved image into a target model.

The to-be-retrieved image may be a two-dimensional image, a three-dimensional image, a pseudo three-dimensional image, or the like. This is not limited.

In an example, the to-be-retrieved image may be a medical image (for example, magnetic resonance imaging, computed tomography imaging, or ultrasound imaging). For example, the to-be-retrieved image may be a lung CT image or a skin image.

For example, before the to-be-retrieved image is inputted into the target model, the to-be-retrieved image may be preprocessed. The preprocessing includes but is not limited to an operation such as image channel adjustment, image scaling, size adjustment, cropping, denoising, rotation transformation (for example, mirror transformation or small-angle rotation), image enhancement, non-target region exclusion, or normalization. For example, an appropriate preprocessing operation may be performed based on an attribute of the to-be-retrieved image. For example, the to-be-retrieved image is a skin image. The preprocessing operation such as image scaling, image normalization, or image channel quantity transformation may be performed on the skin image.

The target model may include a first neural network and a second neural network. The first neural network is a pre-trained model, may also be referred to as a target region segmentation network, and is configured to perform feature extraction on the to-be-retrieved image, and segment a target region based on an extracted feature. For example, the first neural network may be a neural network having an image feature extraction function, for example, a convolutional neural network, a graph convolutional neural network, or a recurrent neural network. For example, the first neural network may be a fully convolutional network (Fully Convolutional Network, FCN), a U-Net network, a generative adversarial network (Generative Adversarial Network, GAN), or a DeepLab series network. The second neural network is a pre-trained model, may also be referred to as a similarity retrieval network, and is configured to perform feature extraction on the to-be-retrieved image, and perform similarity retrieval based on an extracted feature. For example, the second neural network may be a network having an image feature extraction function, for example, a convolutional neural network, a graph convolutional neural network, or a recurrent neural network. For example, the second neural network may be a residual network (residual network, ResNet) or a dense convolutional network (Dense Convolutional Network, DenseNet) network.

S402: Segment the target region in the to-be-retrieved image through the first neural network, to obtain at least one first feature.

For example, the target region may be a region of interest of a user, and there may be one or more target regions. In other words, the to-be-retrieved image may include one or more target regions. In an example, the to-be-retrieved image is a medical image. The target region may be a lesion region in the medical image.

The first feature is an intermediate feature extracted in a process of segmenting the target region. It may be understood that the intermediate feature may be represented in a form of a feature map, a feature vector, or a feature matrix. For example, the first neural network may include one or more convolutional layers. The to-be-retrieved image is inputted into the first neural network, and each convolutional layer in the first neural network may extract a feature of the to-be-retrieved image. A feature of the to-be-retrieved image that is extracted by any convolutional layer is the intermediate feature extracted in the process of segmenting the target region in the to-be-retrieved image through the first neural network. It may be understood that a segmentation result of the target region may be further obtained by segmenting the target region in the to-be-retrieved image through the first neural network.

S403: Process the to-be-retrieved image and the at least one first feature through the second neural network, to obtain a feature corresponding to the target region; and generate, based on the feature corresponding to the target region, target code corresponding to the to-be-retrieved image.

For example, the to-be-retrieved image is inputted into the second neural network. In a process of processing the to-be-retrieved image, the second neural network obtains different features of the to-be-retrieved image through extraction, and fuses the extracted features and the at least one first feature to finally obtain the feature corresponding to the target region. Optionally, a fusion manner may be combination processing, splicing processing, or another processing process used to implement feature fusion. This is not limited in this application.

For example, the feature corresponding to the target region may be a one-dimensional feature. It may be understood that a larger quantity of bits of the one-dimensional feature indicates a stronger capability of representing the target region. The quantity of bits of the one-dimensional feature may be set based on an actual requirement. This is not limited. For example, an operation such as full connection or downsampling may be performed on the extracted intermediate feature, to obtain the one-dimensional feature.

In this step, the second neural network processes the to-be-retrieved image and the at least one first feature, so that the first feature extracted by the first neural network is shared with the second neural network. The first feature is the intermediate feature extracted in the process of segmenting the target region in the to-be-retrieved image through the first neural network; and the first feature includes information about the target region. Therefore, in a process of processing the to-be-retrieved image, the second neural network fuses an extracted feature and the first feature by using the first feature transferred by the first neural network as a guide of feature extraction, so that the intermediate feature extracted in the process of segmenting the target region in the to-be-retrieved image through the first neural network is fully used, to obtain a feature including more accurate information about the target region. For example, the first feature may include semantic information of the target region. In a process of performing feature extraction on the to-be-retrieved image, the second neural network fuses an extracted feature and the first feature, to obtain a feature including more accurate semantic information of the target region.

In an example, the second neural network may include a plurality of convolutional layers and a fully connected layer. The to-be-retrieved image is inputted into the second neural network, convolution operations of the convolutional layers in the second neural network are sequentially performed, different features of the to-be-retrieved image may be extracted, and the feature corresponding to the target region is finally obtained through the fully connected layer. In this process, features extracted by some or all convolutional layers in the second neural network each may be fused with the at least one first feature, to obtain features corresponding to the convolutional layers (if a feature extracted by a convolutional layer is not fused with the at least one first feature, the feature extracted by the convolutional layer is a feature corresponding to the convolutional layer). Therefore, a feature corresponding to each convolutional layer is inputted into a next convolutional layer for a convolution operation, and a feature corresponding to a last convolutional layer is inputted into the fully connected layer for further processing, to obtain the feature corresponding to the target region.

For example, the feature corresponding to the target region may be encoded in an encoding manner such as hash (Hash) encoding or one-hot (one-hot) encoding, to generate the target code. In an example, the target code may be one-dimensional code, for example, one-dimensional hash code. L2 regularization (Regularization) processing may be performed on the feature corresponding to the target region, and then the one-dimensional hash code corresponding to the to-be-retrieved image is generated through hash encoding.

In a possible implementation, the second neural network may be the convolutional neural network. This step of processing the to-be-retrieved image and the at least one first feature through the second neural network, to obtain a feature corresponding to the target region may include: obtaining a first feature that is in the at least one first feature and that corresponds to at least one convolutional layer in the second neural network; fusing a feature extracted by the at least one convolutional layer and the first feature corresponding to the at least one convolutional layer, to obtain a feature corresponding to the at least one convolutional layer; and processing the feature corresponding to the at least one convolutional layer, to obtain the feature corresponding to the target region.

A size of the first feature corresponding to the at least one convolutional layer is the same as a size of the feature extracted by the at least one convolutional layer. The feature extracted by the convolutional layer is a feature extracted by the convolutional layer in the process of processing the to-be- retrieved image through the second neural network. It may be understood that different convolutional layers in the convolutional neural network may extract features of different sizes. For example, when a size of a feature extracted by a convolutional layer is small, more detailed information such as an edge, a location, a color, and a gradient may be included; or when a size of a feature extracted by a convolutional layer is large, stronger semantic information may be included. Features of a same size may include same or similar information. Therefore, in the process of processing the to-be-retrieved image through the second neural network, a first feature that has a same size as a feature extracted by a convolutional layer in the second neural network is transferred to the convolutional layer; and the feature extracted by the convolutional layer is fused with the first feature, and a feature obtained through fusion may be used as a feature corresponding to the convolutional layer. Therefore, feature extraction performance of the convolutional layer on the target region in the to-be-retrieved image is enhanced. Further, the feature corresponding to the convolutional layer is inputted into a next convolutional layer, and the next convolutional layer performs a convolution operation to extract a new feature. In this way, convolution operations of the convolutional layers in the second neural network are sequentially performed on the to-be-retrieved image to finally obtain the feature corresponding to the target region. The obtained feature corresponding to the target region can better represent the target region.

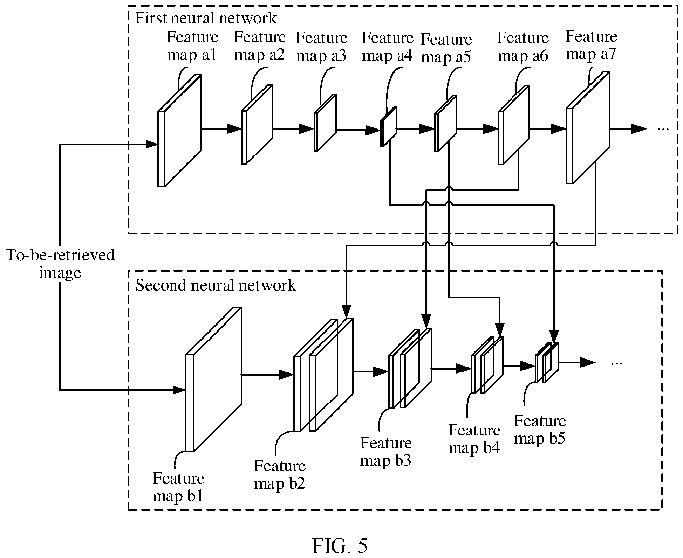

For example, both the first neural network and the second neural network are convolutional neural networks. FIG. 5 is a diagram of transferring a feature between a first neural network and a second neural network according to an embodiment of this application. As shown in FIG. 5, a to-be-retrieved image is inputted into the first neural network and the second neural network. A feature map a1, a feature map a2, a feature map a3, a feature map a4, a feature map a5, a feature map a6, and a feature map a7 are respectively feature maps extracted by different convolutional layers in the first neural network. A feature map b1, a feature map b2, a feature map b3, a feature map b4, and a feature map b5 are respectively feature maps extracted by different convolutional layers in the second neural network. A size of the feature map a4 is the same as a size of the feature map b5, a size of the feature map a5 is the same as a size of the feature map b4, a size of the feature map a6 is the same as a size of the feature map b3, and a size of the feature map a7 is the same as a size of the feature map b2. The first neural network separately transfers the feature map a4, the feature map a5, the feature map a6, and the feature map a7 to the second neural network. The feature map a7 is transferred to a convolutional layer for extracting the feature map b2 in the second neural network, and the feature map b2 is fused with the feature map a7 to obtain a feature corresponding to the convolutional layer. The feature map a6 is transferred to a convolutional layer for extracting the feature map b3 in the second neural network, and the feature map b3 is fused with the feature map a6 to obtain a feature corresponding to the convolutional layer. By analogy, the feature map a4, the feature map a5, the feature map a6, and the feature map a7 are respectively transferred to convolutional layers corresponding to feature maps having a same size as the feature map a4, the feature map a5, the feature map a6, and the feature map a7, that is, convolutional layers for extracting the feature map b5, the feature map b4, the feature map b3, and the feature map b2 in the second neural network. Therefore, a feature map extracted by a convolutional layer in the first neural network is transferred to the second neural network, a convolutional layer in the second neural network fuses an extracted feature map and the feature map transferred by the first neural network. A feature obtained through fusion includes more accurate information about the target region.

S404: Retrieve a target image from a preset image set based on the target code.

The preset image set includes a plurality of images and code corresponding to each of the plurality of images. For example, the code corresponding to each image may be pre-generated, and an encoding manner used to generate the code corresponding to each image is the same as an encoding manner used to generate the target code.

A similarity between code corresponding to the target image and the target code meets a preset condition. For example, the target code may be compared with the code corresponding to each image in the preset image set, and an image corresponding to code with a highest similarity to the target code is determined as the target image. In an example, a distance (for example, a Euclidean distance) between two pieces of code may be used to represent a similarity between two corresponding images. A larger distance between the two pieces of code indicates a lower similarity between the two corresponding images. A smaller distance between the two pieces of code indicates a higher similarity between the two corresponding images. In this case, an image corresponding to code with a minimum distance from the target code in the preset image set may be determined as the target image, to complete similarity retrieval.

For example, a quantity of target images is not limited. In other words, one or more images that are most similar to the to-be-retrieved image may be retrieved from the preset image set.

Further, after the target image is retrieved, the target image may be displayed for a user to view. For example, the target image may be displayed through a display module configured by the image retrieval apparatus, or the target image may be sent to a separately disposed display apparatus, to display the target image.

In an example, the preset image set may further include: a region segmentation result corresponding to each of the plurality of images. After step S404 is performed, a segmentation result obtained by segmenting the target region in the to-be-retrieved image through the first neural network and a region segmentation result corresponding to the target image may be further displayed.

For example, each image may be processed through the first neural network, to pre-obtain a region segmentation result corresponding to each image.

For example, the target image may be displayed through a display module configured by the image retrieval apparatus, or the target image may be sent to a separately disposed display apparatus, to display the segmentation result obtained by segmenting the target region in the to-be-retrieved image through the first neural network and the region segmentation result corresponding to the retrieved target image, so as to facilitate viewing and comparison by the user.

In this embodiment of this application, the target region in the to-be-retrieved image is segmented through the first neural network, and the intermediate feature (namely, the first feature) extracted in the process of segmenting the target region is transferred to the second neural network, so that the feature extracted by the first neural network is shared with the second neural network. Because the first feature includes information about the target region, in a process of processing the to-be-retrieved image, the second neural network may focus on the target region with reference to the first feature, to eliminate interference from invalid information or interference information of a non-target region. The obtained feature corresponding to the target region can better represent the target region. The accurate target code is generated based on the feature corresponding to the target region, and the accurate target code is used to retrieve the target image. In this way, through information exchange between the first neural network and the second neural network, utilization of a feature extracted by the first neural network is improved, so that the second neural network generates accurate target code, thereby effectively improving accuracy of similarity retrieval. In addition, the target model can complete both segmentation of the target region and retrieval of the target image, so that two functions are implemented by using one model. This is more economical and practical.

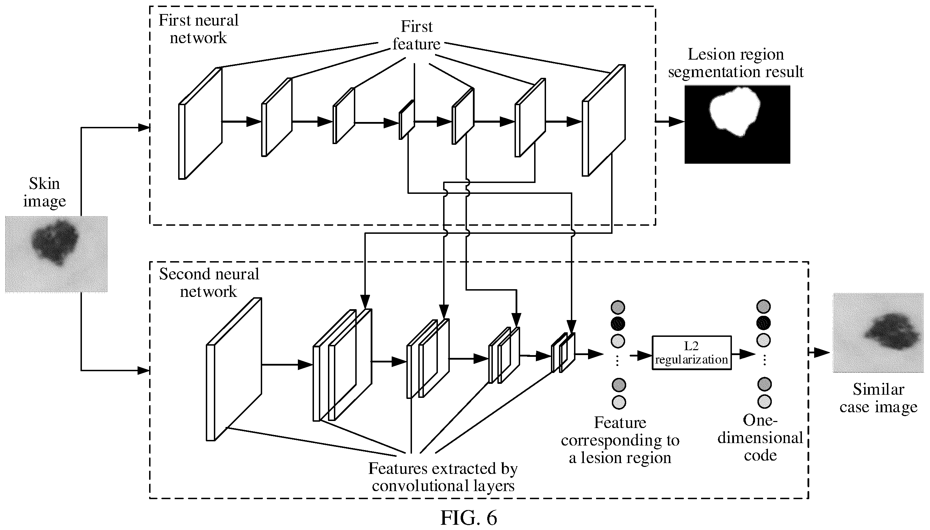

For example, the to-be-retrieved image is a skin image, and the target region is a lesion region of a skin. The retrieved target image may be used as a similar case of the skin image, and is used to provide a reference for subsequent diagnosis performed by a doctor. FIG. 6 is a diagram of an image retrieval method according to an embodiment of this application. As shown in FIG. 6, a skin image is inputted into a first neural network and a second neural network. The first neural network segments a lesion region in the skin image, to generate a plurality of first features and a lesion region segmentation result. A first feature corresponding to a convolutional layer in the second neural network is transferred to the corresponding convolutional layer. In a process of processing the skin image through the second neural network, the convolutional layer that receives the first feature transferred by the first neural network fuses a feature extracted by the convolutional layer and the received first feature, to obtain a feature corresponding to the convolutional layer; and a convolutional layer that does not receive the first feature transferred by the first neural network uses a feature extracted by the convolutional layer as a feature corresponding to the convolutional layer. In this way, convolution operations of convolutional layers are sequentially performed on the skin image to finally obtain a feature corresponding to the lesion region, L2 regularization processing is performed on the feature, and encoding is further performed to generate one-dimensional code corresponding to the skin image. Then, a skin disease database is queried for one-dimensional code of a previously confirmed skin case image. If a similarity between the one-dimensional code corresponding to the skin image and one-dimensional code of a skin case image is the highest, the skin case image is the most similar skin case image, so that similar cases retrieval is completed. The retrieved similar case image can provide a reference for subsequent diagnosis and treatment of a doctor, to improve diagnosis efficiency of the doctor. Therefore, through information exchange between the first neural network and the second neural network, interference from irrelevant information of a non-lesion region to similar cases retrieval is eliminated, and utilization of a feature extracted by the first neural network is improved, so that the second neural network generates accurate one-dimensional code. This effectively improves accuracy of similar cases retrieval. In addition, the target model can complete both segmentation of the lesion region and similar cases retrieval, so that two functions are implemented by using one model. Further, a result of segmenting the lesion region in the skin image through the first neural network and a lesion region segmentation result corresponding to the skin case image in the skin disease database may be further displayed, to facilitate viewing and comparison by the doctor.

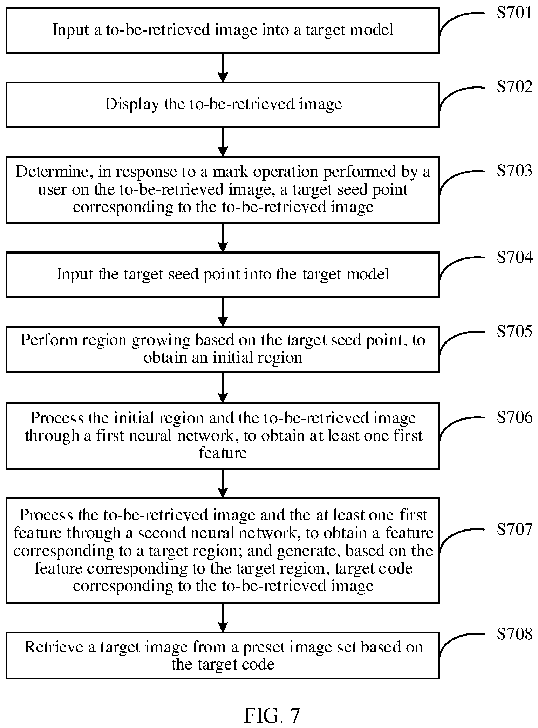

FIG. 7 is a flowchart of an image retrieval method according to an embodiment of this application. The method may be performed by the foregoing image retrieval apparatus. As shown in FIG. 7, the method may include the following steps.

S701: Input a to-be-retrieved image into a target model.

This step is the same as step S401 in FIG. 4. Details are not described herein again.

S702: Display the to-be-retrieved image.

For example, the to-be-retrieved image may be displayed through a display module configured by the image retrieval apparatus, or the to-be-retrieved image may be sent to a separately disposed display apparatus, to display the to-be-retrieved image, so as to facilitate viewing by a user.

S703: Determine, in response to a mark operation performed by a user on the to-be-retrieved image, a target seed point corresponding to the to-be-retrieved image.

The target seed point represents a point in a region of interest of the user. It may be understood that there may be one or more target seed points. For example, the user may perform, based on a region of interest, a mark operation on the displayed to-be-retrieved image in a manner of clicking, selecting, drawing a circle, or the like, so that one or more target seed points are determined in response to the mark operation performed by the user.

In an example, the to-be-retrieved image may be displayed on a touchscreen, and the user may mark the to-be-retrieved image on the touchscreen, and manually select the target seed point.

S704: Input the target seed point into the target model.

For example, the target model may further include a region growing module. The target seed point marked by the user may be inputted into the region growing module.

S705: Perform region growing based on the target seed point, to obtain an initial region.

For example, starting from the target seed point, the region growing module may combine, into a same region, adjacent pixels that are in the to-be-retrieved image and that have attributes similar to those of the target seed point, for example, have similar strength, grayscale, texture, color, and the like to those of the target seed point, to obtain the initial region. It may be understood that the initial region may be used as a coarse segmentation result of a target region.

In an example, the to-be-retrieved image is a medical image, and the region of interest of the user is a lesion region. A coarse segmentation result of the lesion region is obtained through region growing based on the target seed point marked by the user.

For example, the initial region may alternatively be used as a label corresponding to the to-be-retrieved image, and the to-be-retrieved image is used as a training sample, so that the target model is further trained and enhanced, thereby improving performance of the target model.

S706: Segment the initial region and a target region in the to-be-retrieved image through the first neural network, to obtain at least one first feature.

In this step, the first neural network may segment the target region in the to-be-retrieved image with reference to the coarse segmentation result of the target region, to obtain the at least one first feature. For example, a segmentation result of the target region may be further obtained.

In an example, the first neural network may segment the target region in the to-be-retrieved image, and fuse a segmented region and the initial region, to obtain the segmentation result of the target region and the at least one first feature.

It may be understood that the initial region obtained through region growing may be used as the coarse segmentation result of the target region. In this way, in a process of segmenting the target region in the to-be-retrieved image through the first neural network, the coarse segmentation result may be used as a reference, thereby further improving accuracy of the obtained segmentation result of the target region. Correspondingly, the first feature obtained in the process of segmenting the target region may include more accurate information about the target region.

S707: Process the to-be-retrieved image and the at least one first feature through the second neural network, to obtain a feature corresponding to the target region; and generate, based on the feature corresponding to the target region, target code corresponding to the to-be-retrieved image.

This step is the same as step S403 in FIG. 4. Details are not described herein again.

S708: Retrieve a target image from a preset image set based on the target code.

This step is the same as step S404 in FIG. 4. Details are not described herein again.

In this embodiment of this application, the target model obtains the to-be-retrieved image and the target seed point selected by the user, may perform region growing based on the target seed point selected by the user, and in the process of segmenting the target region in the to-be-retrieved image through the first neural network, uses the initial region obtained through region growing as a reference. This further improves accuracy of the obtained segmentation result of the target region. Correspondingly, the intermediate feature extracted in the process of segmenting the target region through the first neural network may include more accurate information about the target region, and the intermediate feature is transferred to the second neural network, so that the second neural network generates more accurate target code. This further improves the accuracy of similarity retrieval.

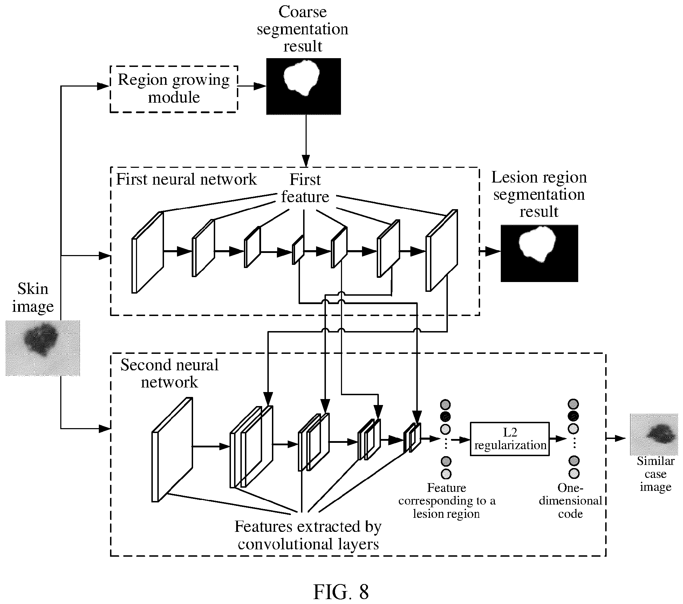

For example, the to-be-retrieved image is a skin image, and the target region is a lesion region of a skin. FIG. 8 is a diagram of an image retrieval method according to an embodiment of this application. As shown in FIG. 8, a skin image is inputted into a region growing module, a first neural network, and a second neural network. The region growing module is configured to perform region growing based on a target seed point selected by a doctor, to obtain a coarse segmentation result of a lesion region, and transfer the coarse segmentation result to the first neural network. The first neural network segments the lesion region in the skin image with reference to the coarse segmentation result, to generate a plurality of first features and an accurate segmentation result of the lesion region. A first feature corresponding to a convolutional layer in the second neural network is transferred to the corresponding convolutional layer. In a process of processing the skin image through the second neural network, the convolutional layer that receives the first feature transferred by the first neural network fuses a feature extracted by the convolutional layer and the received first feature, to obtain a feature corresponding to the convolutional layer; and a convolutional layer that does not receive the first feature transferred by the first neural network uses a feature extracted by the convolutional layer as a feature corresponding to the convolutional layer. In this way, convolution operations of convolutional layers are sequentially performed on the skin image to finally obtain a feature corresponding to the lesion region, L2 regularization processing is performed on the feature, and encoding is further performed to generate one-dimensional code corresponding to the skin image. Then, a skin disease database is queried for one-dimensional code of a previously confirmed skin case image. If a similarity between the one-dimensional code corresponding to the skin image and one-dimensional code of a skin case image is the highest, the skin case image is the most similar skin case image, so that similar cases retrieval is completed, and the retrieved similar case image (namely, the skin case image) can provide a reference for subsequent diagnosis and treatment of a doctor. In this way, region growing is performed based on the target seed point selected by the user, and in the process of segmenting the lesion region in the skin image through the first neural network, the coarse segmentation result of the lesion region obtained through region growing is used as a reference, thereby further improving accuracy of the obtained segmentation result of the lesion region. Correspondingly, an intermediate feature extracted in the process of segment the lesion region through the first neural network may include more accurate information about the lesion region, and the intermediate feature is transferred to the second neural network, so that the second neural network generates more accurate target code. This further improves the accuracy of similar cases retrieval. Further, one or more of the coarse segmentation result, a result of segmenting the lesion region in the skin image through the first neural network, or a lesion region segmentation result corresponding to the skin case image in the skin disease database may be further displayed, to facilitate viewing and comparison by the doctor.

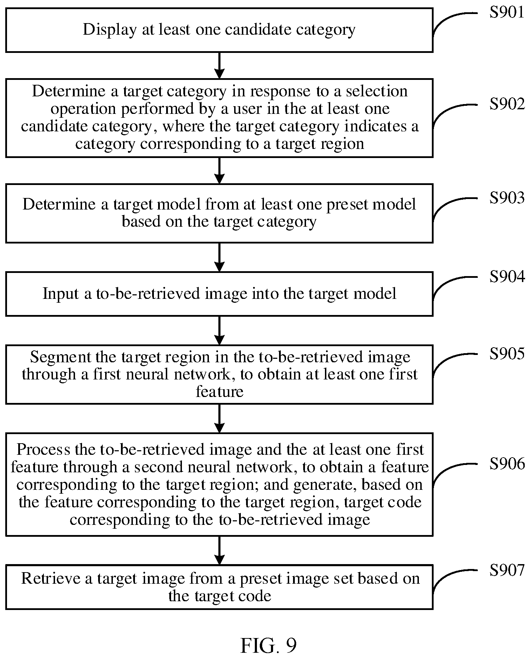

FIG. 9 is a flowchart of an image retrieval method according to an embodiment of this application. The method may be performed by the foregoing image retrieval apparatus. As shown in FIG. 9, the method may include the following steps.

S901: Display at least one candidate category.

A quantity of candidate categories may be set based on a requirement.

For example, the at least one candidate category may be displayed through a display module configured by the image retrieval apparatus, or the at least one candidate category may be sent to a separately disposed display apparatus, to display the at least one candidate category.

In an example, the candidate category may include different human body parts such as a brain, a skin, a lung, and a liver.

S902: Determine a target category in response to a selection operation performed by a user in the at least one candidate category.

The target category represents a category corresponding to a target region, that is, a category on which the user expects to perform similarity retrieval.

In an example, the user may select at least one category from the candidate category as the target category based on a human body part in which a lesion region is located. For example, the target region is a lesion region of a brain. The user may select the brain from the candidate category as the target category.

S903: Determine a target model from at least one preset model based on the target category.

For example, the preset model may be a pre-trained model used for similarity retrieval. The preset model may one to one correspond to the candidate category, in other words, each candidate category corresponds to a preset model obtained through training based on a sample of the candidate category.

In an example, the preset model may include a model used to perform similarity retrieval on a lesion region of a brain, a model used to perform similarity retrieval on a lesion region of a skin, a model used to perform similarity retrieval on a lesion region of a lung, a model used to perform similarity retrieval on a lesion region of a liver, and the like, and the models respectively correspond to different candidate categories such as the brain, the skin, the lung, and the liver. A target model corresponding to a target category may be determined based on the target category selected by the user from the different candidate categories. For example, if the target category selected by the user is the brain, the model used to perform similarity retrieval on the lesion region of the brain is used as the target model.

S904: Input a to-be-retrieved image into the target model.

S905: Segment a target region in the to-be-retrieved image through a first neural network, to obtain at least one first feature.

For example, step S702 to step S706 in FIG. 7 may be performed, so that the initial region is obtained through region growing based on the target seed point marked by the user, and the initial region and the target region in the to-be-retrieved image are segmented through the first neural network, to obtain the at least one first feature.

S906: Process the to-be-retrieved image and the at least one first feature through a second neural network, to obtain a feature corresponding to the target region; and generate, based on the feature corresponding to the target region, target code corresponding to the to-be-retrieved image.

S907: Retrieve a target image from a preset image set based on the target code.

Step S904 to step S907 are respectively the same as step S401 to step S404 in FIG. 4. Details are not described herein again.

In this embodiment of this application, through interaction, the user is allowed to select, from candidate categories, a category on which similarity retrieval is expected to be performed. This improves an interaction capability of the user, so that the user can dynamically adjust the target category based on a requirement, and implement similarity retrieval through a corresponding target model. Therefore, a problem in a related technology that similarity retrieval can be performed only on a single fixed target category is resolved, and practicability is higher.

For example, in the related technology, similarity retrieval can be performed only on a lesion region of a single and fixed part. For example, for a medical image including a lung and a liver, similarity retrieval can be performed only on a lesion region of the lung, and a category on which similarity retrieval is performed cannot be flexibly adjusted based on a clinical requirement. For example, switching between performing similarity retrieval on the lesion region of the lung and performing similarity retrieval on a lesion region of the liver cannot be performed, resulting in limited clinical practicability. In this embodiment of this application, a doctor may be allowed to manually select a category on which similarity retrieval is expected to be performed. For example, when selecting the lung as the target category, the doctor may invoke a model for performing similarity retrieval on the lesion region of the lung to process the medical image, to implement similarity retrieval on the lesion region of the lung. When selecting the liver as the target category, the doctor may invoke a model for performing similarity retrieval on the lesion region of the liver to process the medical image, to implement similarity retrieval on the lesion region of the liver. This improves a capability of interacting with the doctor, so that the doctor can dynamically adjust, based on a requirement, a category on which similarity retrieval is expected to be performed, and clinical practicability is higher.

The foregoing describes the image retrieval method provided in embodiments of this application. The following describes a model training method provided in embodiments of this application from the training side.

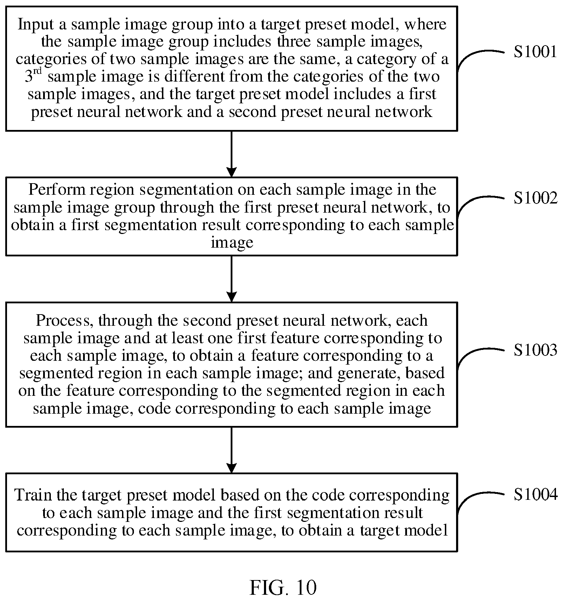

FIG. 10 is a flowchart of a model training method according to an embodiment of this application. The method may be performed by the model training apparatus. As shown in FIG. 10, the method may include the following steps.

S1001: Input a sample image group into a target preset model.

The sample image group includes three sample images, categories of two sample images are the same, and a category of a 3rd sample image is different from the categories of the two sample images. For example, the sample image may be a two-dimensional image, a three-dimensional image, a pseudo three-dimensional image, or the like. This is not limited.

For example, first, a 1st sample image may be randomly selected, as an anchor (Anchor) based on a target category, from a sample image set corresponding to the target category; then, a 2nd sample image is randomly selected from the remaining sample images in the sample image set, or a random transformation operation (for example, rotation or mirror inversion) is performed on the 1st sample image to obtain a 2nd sample image, as a positive sample (Positive sample); and finally, a 3rd sample image is randomly selected, as a negative sample (Negative sample), from sample images corresponding to other categories. In this way, a sample image group is obtained.

In an example, the sample image group may be a medical sample image triplet, including two medical sample images of a same category and one medical sample image of another category. For example, the target category is a skin. A sample image is randomly selected from a sample image set $I = \{I_1, I_2, \cdots, I_T\}$ corresponding to the skin, and is denoted as $\{g1\}$, that is, an anchor; then, a random transformation operation is performed on $\{g1\}$, and a sample image obtained through random transformation is denoted as $\{g2\}$, that is, a positive sample; and finally, a sample image whose category is different from the category of $\{g1\}$ is randomly selected from another sample image set, and a random transformation operation is performed, to obtain a sample image obtained through random transformation, which is denoted as $\{g3\}$, that is, a negative sample. $\{g1, g2, g3\}$ is denoted as a medical sample image triplet.

For example, before the sample image group is inputted into the target preset model, each sample image in the sample image group may be further preprocessed. For a preprocessing manner, refer to the related descriptions of preprocessing the to-be-retrieved image in step S401 in FIG. 4. Details are not described herein again.

The target preset model may include a first preset neural network and a second preset neural network. For example, the first preset neural network may be a common segmentation network, for example, an FCN network, a U-Net network, a GAN network, or a DeepLab series network. For example, the second preset neural network may be a common feature extraction network, for example, a ResNet network or a DenseNet network.

S1002: Perform region segmentation on each sample image in the sample image group through the first preset neural network, to obtain a first segmentation result corresponding to each sample image.

For example, the first preset neural network may separately perform region segmentation on each sample image in the sample image group, that is, sequentially perform region segmentation on each sample image until the three sample images are traversed, and parameters of the first preset neural network are shared when region segmentation is performed on different sample images.

For example, the first segmentation result may include one or more segmented regions. In an example, the sample image is a medical image, and the first segmentation result may include a lesion region in the medical image.

It may be understood that at least one first feature corresponding to each sample image may be further obtained by performing region segmentation on each sample image in the sample image group through the first preset neural network, that is, an intermediate feature extracted in a process of performing region segmentation on each sample image in the sample image group through the first preset neural network.

In an example, the first preset neural network may include one or more convolutional layers. For any sample image in the sample image group, the sample image is inputted into the first preset neural network. Each convolutional layer in the first preset neural network may extract a feature of the sample image. A feature that is of the sample image and that is extracted by any convolutional layer is an intermediate feature extracted in a process of performing region segmentation on the sample image through the first preset neural network.

S1003: Process, through the second preset neural network, each sample image and at least one first feature corresponding to each sample image, to obtain a feature corresponding to a segmented region in each sample image; and generate, based on the feature corresponding to the segmented region in each sample image, code corresponding to each sample image.

For example, the second preset neural network may separately process each sample image in the sample image group, that is, sequentially process each sample image until the three sample images are traversed, and parameters of the second preset neural network are shared when different sample images are processed.

For example, for any sample image in the sample image group, the sample image is inputted into the second preset neural network, and in a process of processing the sample image, the second preset neural network obtains different features of the sample image through extraction, and fuses the extracted features and at least one first feature corresponding to the sample image to finally obtain a feature corresponding to a segmented region in the sample image. For a fusion manner, refer to the foregoing related descriptions.

For example, the feature corresponding to the segmented region in each sample image may be a one-dimensional feature. For a manner of generating the one-dimensional feature, refer to the related descriptions in step S403 in FIG. 4.

For example, for a manner of generating the code corresponding to each sample image, refer to the related descriptions in step S403 in FIG. 4. For example, the code corresponding to each sample image may be one-dimensional code.

In a possible implementation, the second preset neural network may be a convolutional neural network. This step of processing, through the second preset neural network, each sample image and at least one first feature corresponding to each sample image, to obtain a feature corresponding to a segmented region in each sample image may include: obtaining a first feature that is in at least one first feature corresponding to a first sample image and that corresponds to at least one convolutional layer in the second preset neural network; fusing a feature extracted by the at least one convolutional layer in the second preset neural network and the first feature corresponding to the at least one convolutional layer in the second preset neural network, to obtain a feature corresponding to the at least one convolutional layer in the second preset neural network; and processing the feature corresponding to the at least one convolutional layer in the second preset neural network, to obtain a feature corresponding to a segmented region in the first sample image.

The first sample image is any image in the sample image group, and a size of the first feature corresponding to the at least one convolutional layer in the second preset neural network is the same as a size of the feature extracted by at least one convolutional layer in the second preset neural network. Different convolutional layers in the convolutional neural network may extract features of different sizes, and features of a same size may include same or similar information. In a process of processing the first sample image through the second preset neural network, a first feature that corresponds to the first sample image and that has a same size as a feature extracted by a convolutional layer in the second preset neural network is transferred to the convolutional layer; and the feature extracted by the convolutional layer is fused with the first feature, and a feature obtained through fusion may be used as a feature corresponding to the convolutional layer. Therefore, feature extraction performance of the convolutional layer on the segmented region in the first sample image is enhanced. Further, the feature corresponding to the convolutional layer is inputted into a next convolutional layer, and the next convolutional layer performs a convolution operation to extract a new feature. In this way, convolution operations of convolutional layers in the second preset neural network are sequentially performed on the first sample image to finally obtain a feature corresponding to the segmented region in the first sample image. The obtained feature can better represent the segmented region in the first sample image. For example, for an example of transferring a feature between the first preset neural network and the second preset neural network, refer to the related descriptions in FIG. 5.

S 1004: Train the target preset model based on the code corresponding to each sample image and the first segmentation result corresponding to each sample image, to obtain a target model.

For example, the target preset model may be trained based on the code corresponding to each sample image and the first segmentation result corresponding to each sample image until a preset training end condition is met, to obtain the target model. The obtained target model may be used for similarity retrieval on an image of a target category. The preset training end condition may include: A target loss function value no longer decreases or the training reaches a preset quantity of iterations. For example, training of the target preset model may be completed when the training reaches a maximum quantity of iterations, to obtain the target model. For another example, training of the target preset model may be completed when the target loss function value no longer decreases and convergence is implemented, to obtain the target model.

In a possible implementation, this step may include: determining a first loss function value based on the first segmentation result corresponding to each sample image and a label corresponding to each sample image; determining a second loss function value based on the code corresponding to each sample image; determining the target loss function value based on the first loss function value and the second loss function value; and updating the target preset model based on the target loss function value until the preset training end condition is met, to obtain the target model. The label corresponding to each sample image represents an accurate region segmentation result corresponding to each sample image.

In an example, a first loss function may be a dice loss (Dice Loss) function, and the first loss function value may be an average value of dice loss function values corresponding to the three sample images in the sample image group.

For any sample image in the sample image group, a dice loss function value $L1'$ corresponding to the sample image may be calculated by using the following Formula (1):

$$L1' = 1 - \frac{2\sum_{i=1}^{N} P_i G_i}{\sum_{i=1}^{N} P_i^2 + \sum_{i=1}^{N} G_i^2} \tag{1}$$

$P$ is a first segmentation result corresponding to the sample image, $G$ is a label corresponding to the sample image, $N$ is a total quantity of pixels in the sample image, $P_i$ represents a segmentation result corresponding to an $i^{th}$ pixel in the sample image, and $G_i$ represents a label corresponding to the $i^{th}$ pixel in the sample image.

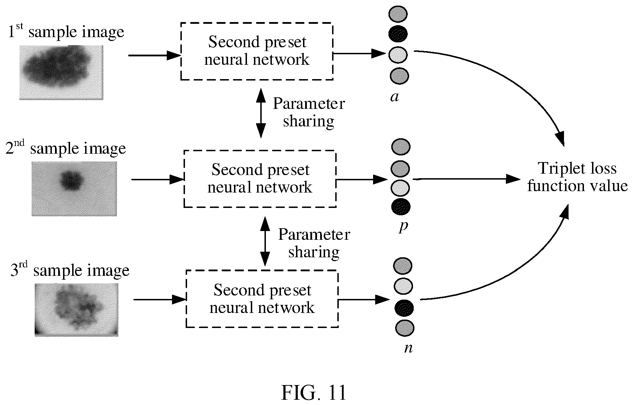

In an example, the second loss function may be a triplet loss (Triplet Loss, TL) function. FIG. 11 is a diagram of calculating a triplet loss function value according to an embodiment of this application. As shown in FIG. 11, a second preset neural network separately processes three sample images in a sample image group, that is, a 1st sample image, a 2nd sample image, and a 3rd sample image, where parameters of the second preset neural network are shared in a process of processing each sample image. One-dimensional code corresponding to each of the three sample images is obtained, that is, code *a*, code *p*, and code *n*. Further, the code *a*, the code *p*, and the code *n* may be calculated by using the following Formula (2), to obtain a triplet loss function value L2.

$$L2 = max\{d(a, p) - d(a, n) + margin, 0\} \qquad (2)$$

*a* represents the code corresponding to the 1st sample image (namely, an anchor) in the sample image group, *p* represents the code corresponding to the 2nd sample image (namely, a positive sample) in the sample image group, *n* represents the code corresponding to the 3rd sample image (namely, a negative sample) in the sample image group, $d(a, p)$ represents a distance (for example, a Euclidean distance) between the code *a* and the code *p*, and $d(a, n)$ represents a distance between the code *a* and the code *n*.

It can be learned from Formula (2) that the triplet loss function value L2 is minimized, so that the distance $d(a, p)$ between the code *a* and the code *p*=0. Because the distance $d(a, n)$ between the code *a* and the code *n* is not less than ($d(a, p) + margin$), when the distance between the code *a* and the code *p* and the distance between *a* and *n* are exactly *margin*, the triplet loss function value L2 is 0; or when the distance between the code *a* and the code *p* and the distance between *a* and *n* are not exactly *margin*, the triplet loss function value is a large value. It may be understood that, a larger value of *margin* indicates a stronger capability of the second preset neural network to distinguish between sample images of different categories, but the network is difficult to train and is not easy to converge; and a smaller value of *margin* indicates a weaker capability of the second preset neural network to distinguish between sample images of different categories, but the network is easy to train. Therefore, the value of *margin* may be set based on specific data. This is not limited.

For example, the first loss function value and the second loss function value may be added as the target loss function value. For example, an average value of dice loss function values corresponding to the three sample images in the sample image group and a corresponding triplet loss function value may be added as the target function loss value.

Further, after the target loss function value is obtained, back propagation may be performed on the target loss function value, and a parameter value in the target preset model is updated by using a gradient descent algorithm, to implement end-to-end (end-to-end) training. Through continuous training, a difference between a segmentation result obtained by performing region segmentation on a sample image through the first preset neural network and a label corresponding to the sample image is gradually narrowed. The second preset neural network continuously performs contrastive learning, so that a similarity (for example, a distance) between code that corresponds to sample images of a same category and that is generated by the second preset neural network is continuously close, and a similarity between generated code corresponding to sample images of different categories continuously increases. In this way, synchronous training is performed on the first preset neural network and the second preset neural network, so that the obtained target model has excellent region segmentation performance and a capability of effectively distinguishing between sample images of different categories.

In this way, the target model corresponding to the target category is obtained by performing step S1001 to step S1004 by using a plurality of sample image groups corresponding to the target category. For example, the target model in FIG. 4, FIG. 7, or FIG. 9 may be obtained. Correspondingly, the trained first preset neural network may be used as the first neural network in FIG. 4, FIG. 7, or FIG. 9, and the trained second preset neural network may be used as the second neural network in FIG. 4, FIG. 7, or FIG. 9. Further, the foregoing step S1001 to step S1004 may be correspondingly performed by using a plurality of sample image groups corresponding to different categories, to obtain preset models corresponding to different categories. For example, the plurality of sample image groups corresponding to different categories may be trained by using a same network structure, to obtain a plurality of corresponding preset models.

Further, after the foregoing training is completed, images of a same category in a database may be sequentially inputted into a preset model corresponding to the category, to generate code corresponding to the image. The code is stored in the database as a basis for subsequent similarity retrieval. For example, the preset model corresponding to the category may further output a region segmentation result corresponding to the image, and may store, in the database, the image of the known category, the code corresponding to the image, and the region segmentation result corresponding to the image as a preset image set.

In an example, a skin image in the database is inputted into a target model obtained through training by using a skin sample image triplet, and a first neural network in the target model segments a lesion region in the skin image, to obtain a lesion region segmentation result. In addition, the first neural network transfers an extracted feature to a second neural network in the target model. The second neural network obtains, through calculation, one-dimensional code corresponding to the skin image, and stores the one-dimensional code in a skin disease database, to provide a basis for subsequent similar cases retrieval. For example, the skin image and the obtained lesion region segmentation result may be further stored in the skin disease database.

In this embodiment of this application, region segmentation is performed on each sample image in the sample image group through the first preset neural network, and the intermediate feature (namely, the first feature corresponding to each sample image) extracted in the process of performing region segmentation is transferred to the second preset neural network, so that the feature extracted by the first preset neural network is shared with the second preset neural network. Because the first feature corresponding to each sample image includes information about the segmented region, in a process of processing each sample image, the second preset neural network may focus on the segmented region with reference to the first feature corresponding to each sample image, to eliminate interference from irrelevant information. The obtained feature corresponding to the segmented region can better represent the segmented region. The code corresponding to each sample image is generated based on the feature corresponding to the segmented region. In this way, in a training process, through information exchange between the first preset neural network and the second preset neural network, utilization of a feature extracted by the first preset neural network is improved, and the code that corresponds to each sample image and that is generated by the second preset neural network through continuous learning can be used to distinguish between sample images of different categories more effectively. Therefore, the target model with excellent similarity retrieval performance is obtained. In addition, in a related technology, in a similarity retrieval manner, a plurality of networks are usually trained in phases, and a training process is complex. In this embodiment of this application, in a training process, information exchange between the first preset neural network and the second preset neural network is added, and the feature extracted by the first preset neural network is transferred to the second preset neural network, so that the first preset neural network and the second preset neural network are no longer independent of each other and are integrated into an entire network (namely, the target preset model), and network training can be performed in an end-to-end manner, to reduce complexity of network training and improve training efficiency.

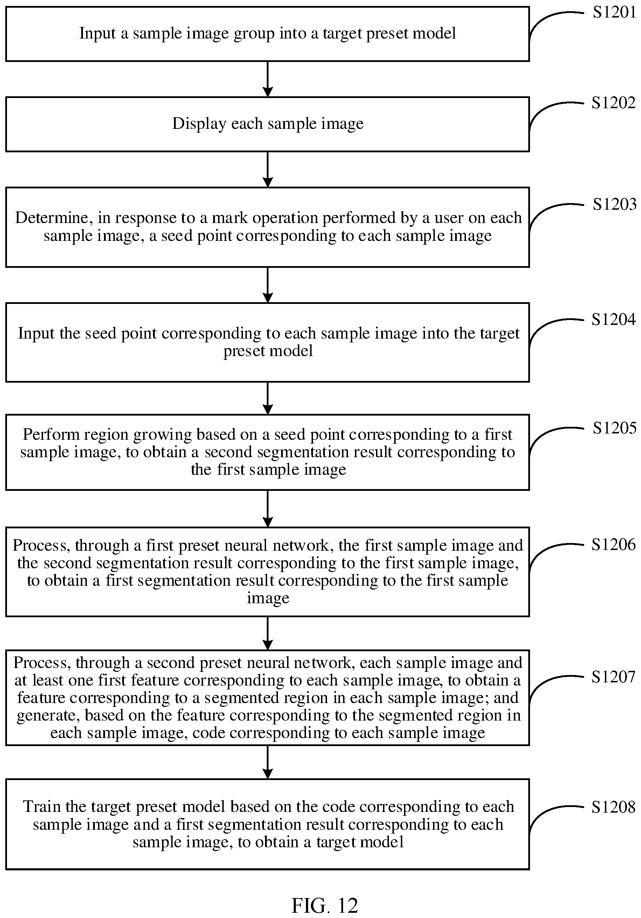

FIG. 12 is a flowchart of another model training method according to an embodiment of this application. The method may be performed by the model training apparatus. As shown in FIG. 12, the method may include the following steps.

S1201: Input a sample image group into a target preset model.

This step is the same as step S1001 in FIG. 10. Details are not described herein again.

S1202: Display each sample image.

For example, each sample image may be displayed through a display module configured by the image retrieval apparatus, or each sample image may be sent to a separately disposed display apparatus, to display each sample image, so as to facilitate viewing by a user.

S1203: Determine, in response to a mark operation performed by a user on each sample image, a seed point corresponding to each sample image.

The seed point represents a point in a region of interest of the user. For example, for a medical image, the seed point may be a point in a lesion region.

In this step, for a possible manner in which the user marks the seed point, refer to the related descriptions of marking the target seed point in step S703 in FIG. 7.

S1204: Input the seed point corresponding to each sample image into the target preset model.

For example, the target preset model may include a region growing module. The seed point marked by the user may be inputted into the region growing module.

It should be noted that step S1202 to step S1204 may alternatively be performed before step S1201. This is not limited.

S1205: Perform region growing based on a seed point corresponding to a first sample image, to obtain a second segmentation result corresponding to the first sample image.

For a manner of implementing region growing, refer to the related descriptions in step S705 in FIG. 7.

In an example, the first sample image is a medical image. The user may mark a seed point in a lesion region in the medical image, and a coarse segmentation result (namely, a second segmentation result) of the lesion region in the first sample image is obtained through region growing based on the seed point marked by the user.

S1206: Process, through a first preset neural network, the first sample image and the second segmentation result corresponding to the first sample image, to obtain a first segmentation result corresponding to the first sample image.

In this step, the first preset neural network may complete region segmentation on the first sample image with reference to the coarse segmentation result of the first sample image, to obtain a more accurate segmentation result (namely, the first segmentation result). It may be understood that at least one first feature corresponding to the first sample image may be further obtained. For example, the first preset neural network may perform region segmentation on the first sample image, and fuse a region segmentation result and the second segmentation result, to obtain the first segmentation result.

S 1207: Process, through a second preset neural network, each sample image and at least one first feature corresponding to each sample image, to obtain a feature corresponding to a segmented region in each sample image; and generate, based on the feature corresponding to the segmented region in each sample image, code corresponding to each sample image.

This step is the same as step S1003 in FIG. 10. Details are not described herein again.

S 1208: Train the target preset model based on the code corresponding to each sample image and the first segmentation result corresponding to each sample image, to obtain a target model.

This step is the same as step S1004 in FIG. 10. Details are not described herein again.

In this way, the target preset model is trained by performing step S 1201 to step S 1208. Region growing is performed based on the seed point selected by the user in each sample image, and in the process of performing region segmentation on each sample image through the first preset neural network, a second segmentation result obtained through region growing is used as a reference. This further improves accuracy of an obtained first segmentation result. Correspondingly, the intermediate feature extracted in the process of performing region segmentation through the first preset neural network may include more accurate information about the segmented region, and the intermediate feature is transferred to the second preset neural network, so that the code generated by the second preset neural network can be used to distinguish between sample images of different categories more effectively. Therefore, performance of the target model obtained through training is further improved.

For example, the target preset model may be trained based on the code corresponding to each sample image, the first segmentation result corresponding to each sample image, and the label corresponding to each sample image, to obtain the target model. The first segmentation result or the second segmentation result corresponding to each sample image may be used as the label corresponding to each sample image.

In a possible implementation, the method may further include: determining a difference between the first segmentation result corresponding to the first sample image and the second segmentation result corresponding to the first sample image; and determining, based on the difference, a label corresponding to the first sample image; and when the difference is greater than a preset value, using the first segmentation result corresponding to the first sample image as the label corresponding to the first sample image, or when the difference is not greater than a preset value, using the second segmentation result corresponding to the first sample image as the label corresponding to the first sample image.

For example, the difference may be represented by using a distance (for example, a Euclidean distance) between the first segmentation result and the second segmentation result. The preset value may be set based on an actual requirement. This is not limited. In the process of training the target preset model, a plurality of rounds of training usually need to be performed. In each round of training, the label corresponding to the first sample image may be determined by comparing the first segmentation result with the second segmentation result. When the difference is greater than the preset value, that is, the difference between the first segmentation result obtained by the first preset neural network and a coarse segmentation result obtained through region growing is large, it indicates that performance of the first preset neural network is greatly improved. In this case, the first segmentation result is used as the label corresponding to the first sample image in the current round of training. When the difference is not greater than the preset value, that is, a difference between the first segmentation result obtained by the first preset neural network and the coarse segmentation result obtained through region growing is small, the coarse segmentation result obtained through region growing is used as the label corresponding to the first sample image in the current round of training. In this case, the coarse segmentation result may be considered as a pseudo label. It may be understood that, in the model training process, the label corresponding to the first sample image is continuously switched between the coarse segmentation result obtained through region growing and the first segmentation result outputted by the first preset neural network, and the difference between the coarse segmentation result and the first segmentation result gradually decreases, and the stable label corresponding to the first sample image may be obtained until the difference can be ignored.

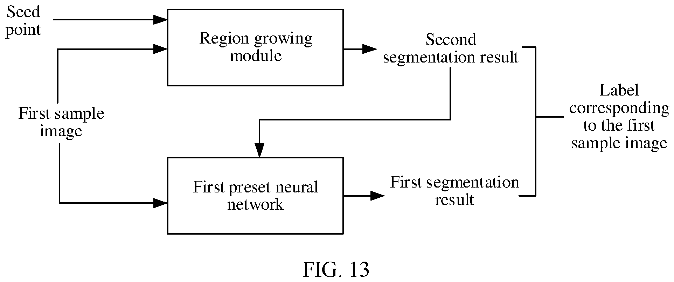

FIG. 13 is a diagram of determining a label corresponding to each sample image according to an embodiment of this application. As shown in FIG. 13, in each round of training, a first sample image and a seed point marked by a user are inputted into a region growing module. The region growing module performs region growing based on the seed point, to obtain a second segmentation result corresponding to the first sample image; and inputs the first sample image and the second segmentation result into a first preset neural network. The first preset neural network outputs a first segmentation result. Further, the first segmentation result may be compared with the second segmentation result. If a difference between the first segmentation result and the second segmentation result is not greater than a preset value, the second segmentation result is used as a pseudo label corresponding to the first sample image, to train the first preset neural network. If the difference between the first segmentation result and the second segmentation result is greater than the preset value, the first segmentation result is used as a label corresponding to the first sample image, to train the first preset neural network, so as to implement semi-supervised learning. In addition, the second segmentation result obtained by performing region growing based on the seed point or the first segmentation result outputted by the first preset neural network is used as the label corresponding to each sample image to train the first preset neural network, and a large quantity of manually marked labels do not need to be obtained, to improve practicability and feasibility of the training method.

In a related technology, in a process of training a segmentation network and a similar cases retrieval network, in addition to a category label on which similar cases retrieval depends, an accurate lesion region segmentation label further needs to be used as a basis for training the segmentation network, and the segmentation label needs to be marked by a professional doctor with a considerable amount of effort, which is time-consuming and labor-intensive. In addition, quality of the label cannot be ensured, and clinical universality is poor. In this embodiment of this application, for a medical sample image, a coarse segmentation result of a lesion region is obtained through region growing based on a seed point marked by a doctor in the lesion region. The coarse segmentation result or a lesion region segmentation result outputted by the first preset neural network is used as a lesion region segmentation label to train the first preset neural network, so as to implement semi-supervised learning of lesion region segmentation. In addition, there is no need to obtain a large quantity of clinically manually marked lesion region segmentation labels, so that a heavy workload of a doctor for manually marking the lesion region in clinic is reduced, thereby improving practicability and feasibility of the training method.

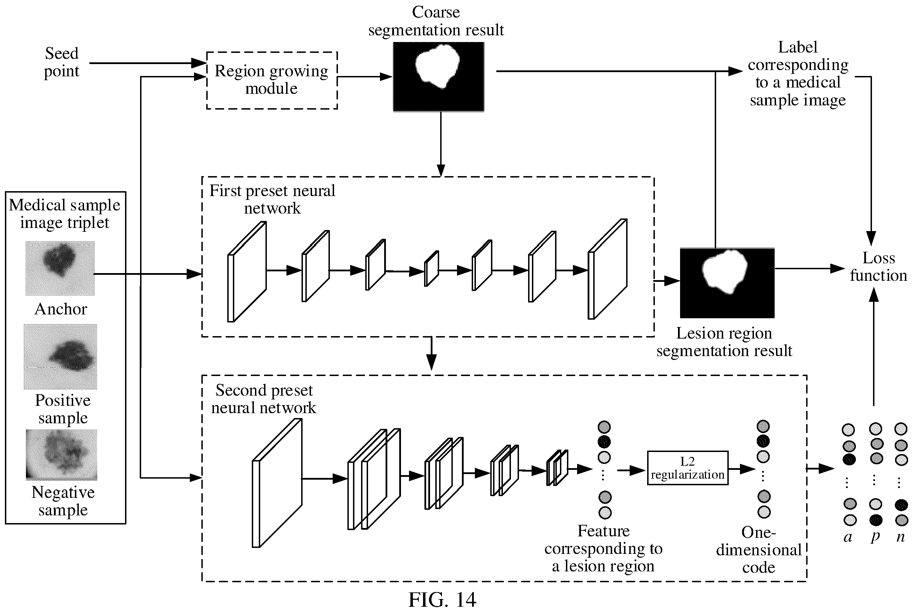

For example, the sample image group is a medical sample image triplet, and both the first preset neural network and the second preset neural network are convolutional neural networks. FIG. 14 is a diagram of a model training method according to an embodiment of this application. As shown in FIG. 14, an anchor in the medical sample image triplet is simultaneously inputted into a region growing module, the first preset neural network, and the second preset neural network. In addition, a seed point marked by a doctor on the anchor is inputted into the region growing module. The region growing module may perform region growing based on the seed point marked by the doctor, to obtain a coarse segmentation result corresponding to the anchor, and may transfer the coarse segmentation result to the first preset neural network. The first preset neural network processes the anchor and the coarse segmentation result, and outputs a lesion region segmentation result corresponding to the anchor. The lesion region segmentation result corresponding to the anchor is compared with the coarse segmentation result that corresponds to the anchor and that is obtained through region growing. If a difference is small, the coarse segmentation result corresponding to the anchor is used as a label corresponding to the anchor. If the difference is large, the lesion region segmentation result corresponding to the anchor is used as the label corresponding to the anchor. The first preset neural network may transfer, to a corresponding convolutional layer in the second preset neural network, a feature extracted by each convolutional layer in a process of performing region segmentation on the anchor. Convolution operations of convolutional layers in the second preset neural network are sequentially performed on the anchor, a one-dimensional feature corresponding to a lesion region in the anchor is finally obtained, L2 regularization processing is performed on the one-dimensional feature, and encoding is further performed to generate one-dimensional code corresponding to the anchor. In this way, with reference to the foregoing processing performed on the anchor, corresponding processing is performed on both a positive sample and a negative sample in the medical sample image triplet. Therefore, the label corresponding to the anchor, a label corresponding to the positive sample, a label corresponding to the negative sample, the lesion region segmentation result corresponding to the anchor, a lesion region segmentation result corresponding to the positive sample, a lesion region segmentation result corresponding to the negative sample, and three pieces of one-dimensional code (namely, one-dimensional code A corresponding to the anchor, one-dimensional code P corresponding to the positive sample, and one-dimensional code N corresponding to the negative sample in the figure) may be obtained, so that a target loss function value may be calculated with reference to Formula (1) and Formula (2), and the target loss function value is used to update the target preset model.

Based on a same inventive concept as the foregoing method embodiments, an embodiment of this application further provides an image retrieval apparatus. The image retrieval apparatus may be configured to perform the technical solutions described in the foregoing method embodiments. For example, the steps of the method shown in FIG. 4, FIG. 7, or FIG. 9 may be performed.

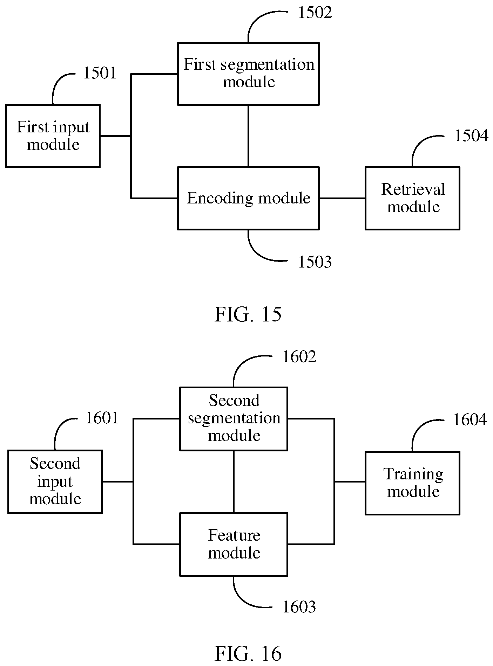

FIG. 15 is a diagram of a structure of an image retrieval apparatus according to an embodiment of this application. As shown in FIG. 15, the apparatus includes: a first input module 1501, configured to input a to-be-retrieved image into a target model, where the target model includes a first neural network and a second neural network; a first segmentation module 1502, configured to segment a target region in the to-be-retrieved image through the first neural network, to obtain at least one first feature, where the first feature is an intermediate feature extracted in a process of segmenting the target region; an encoding module 1503, configured to process the to-be-retrieved image and the at least one first feature through the second neural network, to obtain a feature corresponding to the target region; and generate, based on the feature corresponding to the target region, target code corresponding to the to-be-retrieved image; and a retrieval module 1504, configured to retrieve a target image from a preset image set based on the target code, where the preset image set includes a plurality of images and code corresponding to each of the plurality of images, and a similarity between code corresponding to the target image and the target code meets a preset condition.

In this embodiment of this application, the target region in the to-be-retrieved image is segmented through the first neural network, and the intermediate feature extracted in the process of segmenting the target region is transferred to the second neural network, so that the feature extracted by the first neural network is shared with the second neural network. Because the first feature includes information about the target region, in a process of processing the to-be-retrieved image, the second neural network may focus on the target region with reference to the first feature, to eliminate interference from invalid information or interference information of a non-target region. The obtained feature corresponding to the target region can better represent the target region. The accurate target code is generated based on the feature corresponding to the target region, and the accurate target code is used to retrieve the target image. In this way, through information exchange between the first neural network and the second neural network, utilization of a feature extracted by the first neural network is improved, so that the second neural network generates accurate target code, thereby effectively improving accuracy of similarity retrieval. In addition, the target model can complete both segmentation of the target region and retrieval of the target image, so that two functions are implemented by using one model.

In a possible implementation, the encoding module 1503 is further configured to: obtain a first feature that is in the at least one first feature and that corresponds to at least one convolutional layer in the second neural network, where a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain the feature corresponding to the target region.

In a possible implementation, the apparatus further includes: a first display module, configured to display the to-be-retrieved image; and a marking module, configured to determine, in response to a mark operation performed by a user on the to-be-retrieved image, a target seed point corresponding to the to-be-retrieved image, where the first input module 1501 is further configured to input the target seed point into the target model; and the first segmentation module 1502 is further configured to: perform region growing based on the target seed point, to obtain an initial region; and process the initial region and the to-be-retrieved image through the first neural network, to obtain the at least one first feature.

In a possible implementation, the apparatus further includes: a first display module, configured to display at least one candidate category; a selection module, configured to determine a target category in response to a selection operation performed by the user in the at least one candidate category, where the target category indicates a category corresponding to the target region; and a determining module, configured to determine the target model from at least one preset model based on the target category.

In a possible implementation, the preset image set further includes: a region segmentation result corresponding to each of the plurality of images; and the apparatus further includes: a first display module, configured to display a segmentation result obtained by segmenting the target region and a region segmentation result corresponding to the target image.

For technical effects and specific descriptions of the image retrieval apparatus shown in FIG. 15 and the possible implementations of the image retrieval apparatus, refer to the foregoing image retrieval method. Details are not described herein again.

Based on a same inventive concept as the foregoing method embodiments, an embodiment of this application further provides a model training apparatus. The model training apparatus may be configured to perform the technical solutions described in the foregoing method embodiments. For example, the steps of the method shown in FIG. 10 or FIG. 12 may be performed.

FIG. 16 is a diagram of a structure of a model training apparatus according to an embodiment of this application. As shown in FIG. 16, the apparatus includes: a second input module 1601, configured to input a sample image group into a target preset model, where the sample image group includes three sample images, categories of two sample images are the same, a category of a 3rd sample image is different from the categories of the two sample images, and the target preset model includes a first preset neural network and a second preset neural network; a second segmentation module 1602, configured to perform region segmentation on each sample image in the sample image group through the first preset neural network, to obtain a first segmentation result corresponding to each sample image; a feature module 1603, configured to process, through the second preset neural network, each sample image and at least one first feature corresponding to each sample image, to obtain a feature corresponding to a segmented region in each sample image; and generate, based on the feature corresponding to the segmented region in each sample image, code corresponding to each sample image, where the first feature is an intermediate feature extracted in a process of performing the region segmentation; and a training module 1604, configured to train the target preset model based on the code corresponding to each sample image and the first segmentation result corresponding to each sample image, to obtain a target model.

In this embodiment of this application, region segmentation is performed on each sample image in the sample image group through the first preset neural network, and the intermediate feature extracted in the process of performing region segmentation is transferred to the second preset neural network, so that the feature extracted by the first preset neural network is shared with the second preset neural network. Because the first feature corresponding to each sample image includes information about the segmented region, in a process of processing each sample image, the second preset neural network may focus on the segmented region with reference to the first feature corresponding to each sample image, to eliminate interference from irrelevant information. The obtained feature corresponding to the segmented region can better represent the segmented region. The code corresponding to each sample image is generated based on the feature corresponding to the segmented region. In this way, in a training process, through information exchange between the first preset neural network and the second preset neural network, utilization of a feature extracted by the first preset neural network is improved, and the code that corresponds to each sample image and that is generated by the second preset neural network through continuous learning can be used to distinguish between sample images of different categories more effectively. Therefore, the target model with excellent similarity retrieval performance is obtained. In addition, in a related technology, in a similarity retrieval manner, a plurality of networks are usually trained in phases, and a training process is complex. In this embodiment of this application, in a training process, information exchange between the first preset neural network and the second preset neural network is added, and the feature extracted by the first preset neural network is transferred to the second preset neural network, so that the first preset neural network and the second preset neural network are no longer independent of each other and are integrated into an entire network, and network training can be performed in an end-to-end manner, to reduce complexity of network training and improve training efficiency.

In a possible implementation, the feature module 1603 is further configured to: obtain a first feature that is in at least one first feature corresponding to a first sample image and that corresponds to at least one convolutional layer in the second preset neural network, where the first sample image is any image in the sample image group, and a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain a feature corresponding to a segmented region in the first sample image.

In a possible implementation, the training module 1604 is further configured to: determine a first loss function value based on the first segmentation result corresponding to each sample image and a label corresponding to each sample image; determine a second loss function value based on the code corresponding to each sample image; determine a target loss function value based on the first loss function value and the second loss function value; and update the target preset model based on the target loss function value until a preset training end condition is met, to obtain the target model.

In a possible implementation, the apparatus further includes: a second display module, configured to display each sample image; and a marking module, configured to determine, in response to a mark operation performed by a user on each sample image, a seed point corresponding to each sample image, where the second input module 1601 is further configured to input the seed point corresponding to each sample image into the target preset model; and the second segmentation module 1602 is further configured to: perform region growing based on a seed point corresponding to a first sample image, to obtain a second segmentation result corresponding to the first sample image; and process, through the first preset neural network, the first sample image and the second segmentation result corresponding to the first sample image, to obtain a first segmentation result corresponding to the first sample image.

In a possible implementation, the apparatus further includes: a difference determining module, configured to determine a difference between the first segmentation result corresponding to the first sample image and the second segmentation result corresponding to the first sample image; and a label determining module, configured to determine, based on the difference, a label corresponding to the first sample image; and when the difference is greater than a preset value, use the first segmentation result corresponding to the first sample image as the label corresponding to the first sample image, or when the difference is not greater than a preset value, use the second segmentation result corresponding to the first sample image as the label corresponding to the first sample image.

For technical effects and specific descriptions of the model training apparatus shown in FIG. 16 and the possible implementations of the model training apparatus, refer to the foregoing model training method. Details are not described herein again.

It should be understood that division of the modules in the foregoing image retrieval apparatus and model training apparatus is merely logical function division. During actual implementation, all or some of the modules may be integrated into one physical entity, or may be physically separated. In addition, the modules in the apparatuses may be implemented in a form of software invoked by a processor. For example, the apparatus includes a processor. The processor is connected to a memory. The memory stores instructions. The processor invokes the instructions stored in the memory, to implement any one of the foregoing methods or implement functions of each module of the apparatus. The processor is, for example, a general-purpose processor, for example, a central processing unit (Central Processing Unit, CPU) or a microprocessor. The memory is a memory inside the apparatus or a memory outside the apparatus. Alternatively, the modules in the apparatus may be implemented in a form of hardware circuits, and functions of some or all modules may be implemented by designing the hardware circuits. The hardware circuits may be understood as one or more processors. For example, in an implementation, the hardware circuit is an application-specific integrated circuit (application-specific integrated circuit, ASIC), and the functions of some or all of the foregoing modules are implemented by designing a logical relationship between elements in the circuit. For another example, in another implementation, the hardware circuit may be implemented by using a programmable logic device (programmable logic device, PLD). A field programmable gate array (Field Programmable Gate Array, FPGA) is used as an example, and the field programmable gate array may include a large quantity of logic gate circuits. A configuration file is used to configure a connection relationship between logic gate circuits, so as to implement functions of some or all of the foregoing modules. All the modules of the foregoing apparatuses may be implemented in a form of software invoked by the processor, or all the modules may be implemented in a form of the hardware circuit, or some modules may be implemented in a form of software invoked by the processor, and a remaining part may be implemented in a form of the hardware circuit.

In this embodiment of this application, the processor is a circuit having a signal processing capability. In an implementation, the processor may be a circuit having an instruction reading and running capability, for example, a CPU, a microprocessor, a graphics processing unit (graphics processing unit, GPU), a digital signal processor (digital signal processor, DSP), a neural-network processing unit (neural-network processing unit, NPU), or a tensor processing unit (tensor processing unit, TPU). In another implementation, the processor may implement a specific function by using the logical relationship of the hardware circuit. The logical relationship of the hardware circuit is fixed or reconfigurable. For example, the processor is a hardware circuit implemented by an ASIC or a PLD, for example, an FPGA. In the reconfigurable hardware circuit, a process in which the processor loads a configuration document to implement hardware circuit configuration may be understood as a process in which the processor loads instructions to implement functions of some or all of the foregoing modules.

It can be learned that each module in the foregoing apparatus may be one or more processors (or processing circuits) configured to implement the method in the foregoing embodiments, for example, a CPU, a GPU, an NPU, a TPU, a microprocessor, a DSP, an ASIC, an FPGA, or a combination of at least two of these processor forms. In addition, all or some of the modules in the foregoing apparatuses may be integrated together, or may be implemented independently. This is not limited.

An embodiment of this application further provides an electronic device, including: a processor, and a memory configured to store instructions executable by the processor. When the processor is configured to execute the instructions, the method in the foregoing embodiments is implemented. For example, the steps of the method shown in FIG. 4, FIG. 7, or FIG. 9 may be performed, or the steps of the method shown in FIG. 10 or FIG. 12 may be performed.

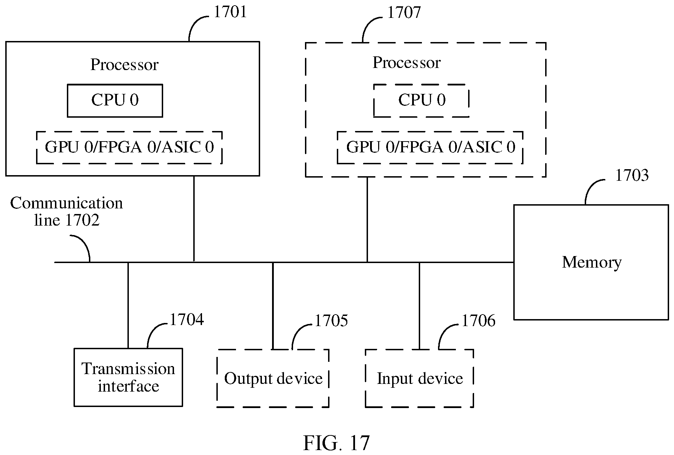

FIG. 17 is a diagram of a structure of an electronic device according to an embodiment of this application. As shown in FIG. 17, the electronic deviceelectronic device may include: at least one processor 1701, a communication line 1702, a memory 1703, and at least one communication interface 704.

The processor 1701 may be a general-purpose central processing unit, a microprocessor, an application-specific integrated circuit, or one or more integrated circuits configured to control program execution of the solutions of this application. The processor 1701 may alternatively include a heterogeneous computing architecture of a plurality of general-purpose processors, for example, may be a combination of at least two of a CPU, a GPU, a microprocessor, a DSP, an ASIC, and an FPGA. In an example, the processor 1701 may be the CPU plus the GPU, the CPU plus the ASIC, or the CPU plus the FPGA.

The communication line 1702 may include a path for information transmission between the foregoing components.

The communication interface 1704 is any apparatus such as a transceiver, and is configured to communicate with another device or communication network such as the Ethernet, a RAN, or a wireless local area network (wireless local area network, WLAN).

The memory 1703 may be a read-only memory (read-only memory, ROM) or another type of static storage device that can store static information and instructions, or a random access memory (random access memory, RAM) or another type of dynamic storage device that can store information and instructions. Alternatively, the memory 1703 may be an electrically erasable programmable read-only memory (electrically erasable programmable read-only memory, EEPROM), a compact disc read-only memory (compact disc read-only memory, CD-ROM) or another compact disc storage, an optical disc storage (including a compact optical disc, a laser disc, an optical disc, a digital versatile disc, a Blu-ray disc, or the like), a magnetic disk storage medium or another magnetic storage device, or any other medium that can be used to carry or store expected program code in a form of an instruction or a data structure and that can be accessed by a computer. However, the memory 1703 is not limited thereto. The memory may exist independently, and is connected to the processor through the communication line 1702. The memory may alternatively be integrated with the processor. The memory provided in this embodiment of this application may be usually non-volatile. The memory 1703 is configured to store computer-executable instructions for executing the solutions of this application, and the processor 1701 controls execution. The processor 1701 is configured to execute the computer-executable instructions stored in the memory 1703, to implement the method provided in the foregoing embodiments of this application. For example, the steps of the method shown in FIG. 4, FIG. 7, or FIG. 9 may be implemented, or the steps of the method shown in FIG. 10 or FIG. 12 may be performed.

Optionally, the computer-executable instructions in this embodiment of this application may also be referred to as application program code. This is not specifically limited in this embodiment of this application.

For example, the processor 1701 may include one or more CPUs, for example, a CPU 0 in FIG. 17. The processor 701 may alternatively include one CPU and any one of a GPU, an ASIC, and an FPGA, for example, the CPU 0 plus a GPU 0, the CPU 0 plus an ASIC 0, or the CPU 0 plus an FPGA 0 in FIG. 17.

For example, the electronic device may include a plurality of processors, for example, the processor 1701 and a processor 1707 in FIG. 17. Each of the processors may be a single-core (single-CPU) processor, may be a multi-core (multi-CPU) processor, or may be a heterogeneous computing architecture including a plurality of general-purpose processors. The processor herein may be one or more devices, circuits, and/or processing cores configured to process data (for example, computer program instructions).

During specific implementation, in an embodiment, the electronic device may further include an output device 1705 and an input device 1706. The output device 1705 communicates with the processor 1701, and may display information in a plurality of manners. For example, the output device 1705 may be a liquid crystal display (liquid crystal display, LCD), a light emitting diode (light emitting diode, LED) display device, a cathode ray tube (cathode ray tube, CRT) display device, or a projector (projector). For example, the output device may be a display device such as a vehicle-mounted HUD, an AR-HUD, or a display. The input device 1706 communicates with the processor 1701, and may receive an input of a user in a plurality of manners. For example, the input device 1706 may be a mouse, a keyboard, a touchscreen device, or a sensor device.

An embodiment of this application provides a computer-readable storage medium storing computer program instructions. When the computer program instructions are executed by a processor, the method in the foregoing embodiments is implemented. For example, the steps of the method shown in FIG. 4, FIG. 7, or FIG. 9 may be implemented, or the steps of the method shown in FIG. 10 or FIG. 12 may be performed.

An embodiment of this application provides a computer program product. For example, the computer program product may include computer-readable code or a non-volatile computer-readable storage medium carrying computer-readable code. When the computer program product runs on a computer, the computer is enabled to perform the method in the foregoing embodiments. For example, the steps of the method shown in FIG. 4, FIG. 7, or FIG. 9 may be performed, or the steps of the method shown in FIG. 10 or FIG. 12 may be performed.

The computer-readable storage medium may be a tangible device that can retain and store instructions for use by an instruction execution device. The computer-readable storage medium may be, for example, but is not limited to, an electrical storage device, a magnetic storage device, an optical storage device, an electromagnetic storage device, a semiconductor storage device, or any suitable combination thereof. A more specific example (a non-exhaustive list) of the computer-readable storage medium includes: a portable computer disk, a hard disk, a random access memory (RAM), a read-only memory (ROM), and an erasable programmable read-only memory (EPROM or flash memory), a static random access memory (SRAM), a portable compact disc read-only memory (CD-ROM), a digital versatile disc (DVD), a memory stick, a floppy disk, a mechanical coding device, for example, a punch card or a protrusion structure in a groove that stores instructions, and any suitable combination of the foregoing devices. The computer-readable storage medium used herein is not to be construed as a transient signal, such as a radio wave or another freely propagating electromagnetic wave, an electromagnetic wave propagating through a waveguide or another transmission medium (such as a light pulse through a fiber optic cable), or an electrical signal transmitted through a wire.

The computer-readable program instructions described herein may be downloaded from the computer-readable storage medium to respective computing/processing devices or to an external computer or external storage device through a network such as the internet, a local area network, a wide area network, and/or a wireless network. The network may include a copper transmission cable, optical fiber transmission, wireless transmission, a router, a firewall, a switch, a gateway computer, and/or an edge server. A network adapter card or a network interface in each computing/processing device receives the computer-readable program instructions from the network, and forwards the computer-readable program instructions for storage in a computer-readable storage medium in each computing/processing device.

The computer program instructions used to perform operations in this application may be an assembly instruction, an instruction set architecture (ISA) instruction, a machine instruction, a machine-related instruction, microcode, a firmware instruction, status setting data, or source code or target code written in any combination of one or more programming languages. The programming languages include an object-oriented programming language such as Smalltalk and C++, and a conventional procedural programming language such as a "C" language or a similar programming language. The computer-readable program instructions may be executed completely on a user computer, executed partially on the user computer, executed as a standalone software package, executed partially on the user computer and partially on a remote computer, or executed completely on the remote computer or a server. When a remote computer is involved, the remote computer may be connected to a user computer through any type of network, including a local area network (LAN) or a wide area network (WAN), or may be connected to an external computer (for example, connected through an internet service provider through the internet). In some embodiments, an electronic circuit, for example, a programmable logic circuit, a field programmable gate array (FPGA), or a programmable logic array (PLA), is customized by using status information of the computer-readable program instructions. The electronic circuit may execute the computer-readable program instructions, so as to implement the various aspects of this application.

The various aspects of this application are described herein with reference to the flowcharts and/or block diagrams of the method, the apparatus (system), and the computer program product according to embodiments of this application. It should be understood that, each block of the flowcharts and/or block diagrams and a combination of blocks in the flowcharts and/or block diagrams may be implemented by the computer-readable program instructions.

These computer-readable program instructions may be provided to a processor of a general-purpose computer, a dedicated computer, or another programmable data processing apparatus to produce a machine, so that the instructions, when executed by the processor of the computer or the another programmable data processing apparatus, create an apparatus for implementing functions/actions specified in one or more blocks in the flowcharts and/or block diagrams. These computer-readable program instructions may alternatively be stored in the computer-readable storage medium. These instructions enable the computer, the programmable data processing apparatus, and/or another device to work in a specific manner. Therefore, the computer-readable medium storing the instructions includes an artifact that includes instructions for implementing various aspects of the functions/actions specified in the one or more blocks in the flowcharts and/or block diagrams.

The computer-readable program instructions may alternatively be loaded onto a computer, another programmable data processing apparatus, or another device, so that a series of operation steps are performed on the computer, the another programmable data processing apparatus, or the another device to produce a computer-implemented process. Therefore, the instructions executed on the computer, the another programmable data processing apparatus, or the another device implement the functions/actions specified in the one or more blocks in the flowcharts and/or block diagrams.

The flowcharts and block diagrams in the accompanying drawings show the system architecture, function, and operation of possible implementations of systems, methods, and computer program products according to various embodiments of this application. In this regard, each block in the flowcharts or block diagrams may represent a module, a program segment, or a part of the instructions, and the module, the program segment, or the part of the instructions includes one or more executable instructions for implementing a specified logical function. In some alternative implementations, a function marked in the block may also occur in a sequence different from that marked in the accompanying drawings. For example, two consecutive blocks may actually be executed substantially in parallel, and may sometimes be executed in a reverse order. This depends on a function involved. It should also be noted that each block in the block diagrams and/or flowcharts, and the combination of the blocks in the block diagrams and/or flowcharts may be implemented by a special-purpose hardware-based system that performs a specified function or act, or may be implemented by a combination of special-purpose hardware and computer instructions.

Embodiments of this application are described above. The foregoing descriptions are examples, are not exhaustive, and are not limited to the disclosed embodiments. Many modifications and changes are apparent to a person of ordinary skill in the art without departing from the scope and spirit of the illustrated embodiments. Selection of terms used in this specification is intended to best explain the principles of embodiments, actual application, or improvements to technologies in the market, or to enable another person of ordinary skill in the art to understand embodiments disclosed in this specification.

## Claims

1. An image retrieval method, comprising:

   inputting a to-be-retrieved image into a target model, wherein the target model comprises a first neural network and a second neural network;
   segmenting a target region in the to-be-retrieved image through the first neural network, to obtain at least one first feature, wherein the first feature is an intermediate feature extracted in a process of segmenting the target region;
   processing the to-be-retrieved image and the at least one first feature through the second neural network, to obtain a feature corresponding to the target region; and generating, based on the feature corresponding to the target region, target code corresponding to the to-be-retrieved image; and
   retrieving a target image from a preset image set based on the target code, wherein the preset image set comprises a plurality of images and code corresponding to each of the plurality of images, and a similarity between code corresponding to the target image and the target code meets a preset condition.

2. The method according to claim 1, wherein the processing the to-be-retrieved image and the at least one first feature through the second neural network, to obtain a feature corresponding to the target region comprises:

   obtaining a first feature that is in the at least one first feature and that corresponds to at least one convolutional layer in the second neural network, wherein a size of the first feature corresponding to the at least one convolutional layer is the same as a size of a feature extracted by the at least one convolutional layer;
   fusing the feature extracted by the at least one convolutional layer and the first feature corresponding to the at least one convolutional layer, to obtain a feature corresponding to the at least one convolutional layer; and
   processing the feature corresponding to the at least one convolutional layer, to obtain the feature corresponding to the target region.

3. The method according to claim 1 or 2, wherein the method further comprises:

> displaying the to-be-retrieved image;
> determining, in response to a mark operation performed by a user on the to-be-retrieved image, a target seed point corresponding to the to-be-retrieved image; and
> inputting the target seed point into the target model; and
> the segmenting a target region in the to-be-retrieved image through the first neural network, to obtain at least one first feature comprises:
>
>> performing region growing based on the target seed point, to obtain an initial region; and
>> segmenting the initial region and the target region in the to-be-retrieved image through the first neural network, to obtain the at least one first feature.

4. The method according to any one of claims 1 to 3, wherein the method further comprises:

> displaying at least one candidate category;
> determining a target category in response to a selection operation performed by the user in the at least one candidate category, wherein the target category indicates a category corresponding to the target region; and
> determining the target model from at least one preset model based on the target category.

5. The method according to any one of claims 1 to 4, wherein the preset image set further comprises: a region segmentation result corresponding to each of the plurality of images; and
the method further comprises:
displaying a segmentation result obtained by segmenting the target region and a region segmentation result corresponding to the target image.

6. A model training method, comprising:

> inputting a sample image group into a target preset model, wherein the sample image group comprises three sample images, categories of two sample images are the same, a category of a 3rd sample image is different from the categories of the two sample images, and the target preset model comprises a first preset neural network and a second preset neural network;
> performing region segmentation on each sample image in the sample image group through the first preset neural network, to obtain a first segmentation result corresponding to each sample image;
> processing, through the second preset neural network, each sample image and at least one first feature corresponding to each sample image, to obtain a feature corresponding to a segmented region in each sample image; and generating, based on the feature corresponding to the segmented region in each sample image, code corresponding to each sample image, wherein the first feature is an intermediate feature extracted in a process of performing the region segmentation; and
> training the target preset model based on the code corresponding to each sample image and the first segmentation result corresponding to each sample image, to obtain a target model.

7. The method according to claim 6, wherein the processing, through the second preset neural network, each sample image and at least one first feature corresponding to each sample image, to obtain a feature corresponding to a segmented region in each sample image comprises:

> obtaining a first feature that is in at least one first feature corresponding to a first sample image and that corresponds to at least one convolutional layer in the second preset neural network, wherein the first sample image is any image in the sample image group, and a size of the first feature corresponding to the at least one convolutional layer is the same as a size of a feature extracted by the at least one convolutional layer;
> fusing the feature extracted by the at least one convolutional layer and the first feature corresponding to the at least one convolutional layer, to obtain a feature corresponding to the at least one convolutional layer; and
> processing the feature corresponding to the at least one convolutional layer, to obtain a feature corresponding to a segmented region in the first sample image.

8. The method according to claim 6 or 7, wherein the training the target preset model based on the code corresponding to each sample image and the first segmentation result corresponding to each sample image, to obtain a target model comprises:

determining a first loss function value based on the first segmentation result corresponding to each sample image and a label corresponding to each sample image;

determining a second loss function value based on the code corresponding to each sample image;

determining a target loss function value based on the first loss function value and the second loss function value; and

updating the target preset model based on the target loss function value until a preset training end condition is met, to obtain the target model.

9. The method according to any one of claims 6 to 8, wherein the method further comprises:

displaying each sample image;

determining, in response to a mark operation performed by a user on each sample image, a seed point corresponding to each sample image; and

inputting the seed point corresponding to each sample image into the target preset model; and

the performing region segmentation on each sample image in the sample image group through the first preset neural network, to obtain a first segmentation result corresponding to each sample image comprises:

performing region growing based on a seed point corresponding to a first sample image, to obtain a second segmentation result corresponding to the first sample image; and

processing, through the first preset neural network, the first sample image and the second segmentation result corresponding to the first sample image, to obtain a first segmentation result corresponding to the first sample image.

10. The method according to claim 9, wherein the method further comprises:

determining a difference between the first segmentation result corresponding to the first sample image and the second segmentation result corresponding to the first sample image; and

determining, based on the difference, a label corresponding to the first sample image; and when the difference is greater than a preset value, using the first segmentation result corresponding to the first sample image as the label corresponding to the first sample image, or when the difference is not greater than a preset value, using the second segmentation result corresponding to the first sample image as the label corresponding to the first sample image.

11. An image retrieval apparatus, comprising: an input module, configured to input a to-be-retrieved image into a target model, wherein the target model comprises a first neural network and a second neural network; a segmentation module, configured to segment a target region in the to-be-retrieved image through the first neural network, to obtain at least one first feature, wherein the first feature is an intermediate feature extracted in a process of segmenting the target region; an encoding module, configured to: process the to-be-retrieved image and the at least one first feature through the second neural network, to obtain a feature corresponding to the target region; and generate, based on the feature corresponding to the target region, target code corresponding to the to-be-retrieved image; and a retrieval module, configured to retrieve a target image from a preset image set based on the target code, wherein the preset image set comprises a plurality of images and code corresponding to each of the plurality of images, and a similarity between code corresponding to the target image and the target code meets a preset condition.

12. The image retrieval apparatus according to claim 11, wherein the encoding module is further configured to: obtain a first feature that is in the at least one first feature and that corresponds to at least one convolutional layer in the second neural network, wherein a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain the feature corresponding to the target region.

13. The image retrieval apparatus according to claim 11 or 12, wherein the apparatus further comprises: a display module, configured to display the to-be-retrieved image; and a marking module, configured to determine, in response to a mark operation performed by a user on the to-be-retrieved image, a target seed point corresponding to the to-be-retrieved image, wherein the input module is further configured to input the target seed point into the target model; and the segmentation module is further configured to: perform region growing based on the target seed point, to obtain an initial region; and process the initial region and the to-be-retrieved image through the first neural network, to obtain the at least one first feature.

14. The image retrieval apparatus according to any one of claims 11 to 13, wherein the apparatus further comprises: a display module, configured to display at least one candidate category; a selection module, configured to determine a target category in response to a selection operation performed by the user in the at least one candidate category, wherein the target category indicates a category corresponding to the target region; and a determining module, configured to determine the target model from at least one preset model based on the target category.

15. The image retrieval apparatus according to any one of claims 11 to 14, wherein the preset image set further comprises: a region segmentation result corresponding to each of the plurality of images; and the apparatus further comprises: a display module, configured to display a segmentation result obtained by segmenting the target region and a region segmentation result corresponding to the target image.

16. A model training apparatus, comprising: an input module, configured to input a sample image group into a target preset model, wherein the sample image group comprises three sample images, categories of two sample images are the same, a category of a $3^{rd}$ sample image is different from the categories of the two sample images, and the target preset model comprises a first preset neural network and a second preset neural network; a segmentation module, configured to perform region segmentation on each sample image in the sample image group through the first preset neural network, to obtain a first segmentation result corresponding to each sample image; a feature module, configured to: process, through the second preset neural network, each sample image and at least one first feature corresponding to each sample image, to obtain a feature corresponding to a segmented region in each sample image; and generate, based on the feature corresponding to the segmented region in each sample image, code corresponding to each sample image, wherein the first feature is an intermediate feature extracted in a process of performing the region segmentation; and a training module, configured to train the target preset model based on the code corresponding to each sample image and the first segmentation result corresponding to each sample image, to obtain a target model.

17. The model training apparatus according to claim 16, wherein the feature module is further configured to: obtain a first feature that is in at least one first feature corresponding to a first sample image and that corresponds to at least one convolutional layer in the second preset neural network, wherein the first sample image is any image in the sample image group, and a size of the first feature corresponding to the at least one convolutional layer is the same as a size of a feature extracted by the at least one convolutional layer; fuse the feature extracted by the at least one convolutional layer and the first feature corresponding to the at least one convolutional layer, to obtain a feature corresponding to the at least one convolutional layer; and process the feature corresponding to the at least one convolutional layer, to obtain a feature corresponding to a segmented region in the first sample image.

18. The model training apparatus according to claim 16 or 17, wherein the training module is further configured to: determine a first loss function value based on the first segmentation result corresponding to each sample image and a label corresponding to each sample image; determine a second loss function value based on the code corresponding to each sample image; determine a target loss function value based on the first loss function value and the second loss function value; and update the target preset model based on the target loss function value until a preset training end condition is met, to obtain the target model.

19. The model training apparatus according to any one of claims 16 to 18, wherein the apparatus further comprises: a display module, configured to display each sample image; and a marking module, configured to determine, in response to a mark operation performed by a user on each sample image, a seed point corresponding to each sample image, wherein the input module is further configured to input the seed point corresponding to each sample image into the target preset model; and the segmentation module is further configured to: perform region growing based on a seed point corresponding to a first sample image, to obtain a second segmentation result corresponding to the first sample image; and process, through the first preset neural network, the first sample image and the second segmentation result corresponding to the first sample image, to obtain a first segmentation result corresponding to the first sample image.

20. The model training apparatus according to claim 19, wherein the apparatus further comprises: a difference determining module, configured to determine a difference between the first segmentation result corresponding to the first sample image and the second segmentation result corresponding to the first sample image; and a label determining module, configured to: determine, based on the difference, a label corresponding to the first sample image; and when the difference is greater than a preset value, use the first segmentation result corresponding to the first sample image as the label corresponding to the first sample image, or when the difference is not greater than a preset value, use the second segmentation result corresponding to the first sample image as the label corresponding to the first sample image.

21. An electronic device, comprising:

a processor; and
a memory configured to store instructions executable by the processor, wherein
the processor is configured to implement the method according to any one of claims 1 to 5 or implement the method according to any one of claims 6 to 10 when executing the instructions.

22. Anon-volatile computer-readable storage medium, storing computer program instructions, wherein when the computer program instructions are executed by a processor, the method according to any one of claims 1 to 5 is implemented, or the method according to any one of claims 6 to 10 is implemented.

FIG. 1

FIG. 2

FIG. 3

Input a to-be-retrieved image into a target model, where the target model includes a first neural network and a second neural network ⟶ S401

Segment a target region in the to-be-retrieved image through the first neural network, to obtain at least one first feature, where the first feature is an intermediate feature extracted in a process of segmenting the target region ⟶ S402

Process the to-be-retrieved image and the at least one first feature through the second neural network, to obtain a feature corresponding to the target region; and generate, based on the feature corresponding to the target region, target code corresponding to the to-be-retrieved image ⟶ S403

Retrieve a target image from a preset image set based on the target code, where the preset image set includes a plurality of images and code corresponding to each of the plurality of images, and a similarity between code corresponding to the target image and the target code meets a preset condition ⟶ S404

FIG. 4

First neural network

Feature map a1  Feature map a2  Feature map a3  Feature map a4  Feature map a5  Feature map a6  Feature map a7

To-be-retrieved image

Second neural network

Feature map b1  Feature map b2  Feature map b3  Feature map b4  Feature map b5

FIG. 5

FIG. 6

Input a to-be-retrieved image into a target model — S701

Display the to-be-retrieved image — S702

Determine, in response to a mark operation performed by a user on the to-be-retrieved image, a target seed point corresponding to the to-be-retrieved image — S703

Input the target seed point into the target model — S704

Perform region growing based on the target seed point, to obtain an initial region — S705

Process the initial region and the to-be-retrieved image through a first neural network, to obtain at least one first feature — S706

Process the to-be-retrieved image and the at least one first feature through a second neural network, to obtain a feature corresponding to a target region; and generate, based on the feature corresponding to the target region, target code corresponding to the to-be-retrieved image — S707

Retrieve a target image from a preset image set based on the target code — S708

FIG. 7

FIG. 8

| Display at least one candidate category | S901 |

↓

| Determine a target category in response to a selection operation performed by a user in the at least one candidate category, where the target category indicates a category corresponding to a target region | S902 |

↓

| Determine a target model from at least one preset model based on the target category | S903 |

↓

| Input a to-be-retrieved image into the target model | S904 |

↓

| Segment the target region in the to-be-retrieved image through a first neural network, to obtain at least one first feature | S905 |

↓

| Process the to-be-retrieved image and the at least one first feature through a second neural network, to obtain a feature corresponding to the target region; and generate, based on the feature corresponding to the target region, target code corresponding to the to-be-retrieved image | S906 |

↓

| Retrieve a target image from a preset image set based on the target code | S907 |

FIG. 9

Input a sample image group into a target preset model, where the sample image group includes three sample images, categories of two sample images are the same, a category of a 3$^{rd}$ sample image is different from the categories of the two sample images, and the target preset model includes a first preset neural network and a second preset neural network ⌐ S1001

Perform region segmentation on each sample image in the sample image group through the first preset neural network, to obtain a first segmentation result corresponding to each sample image ⌐ S1002

Process, through the second preset neural network, each sample image and at least one first feature corresponding to each sample image, to obtain a feature corresponding to a segmented region in each sample image; and generate, based on the feature corresponding to the segmented region in each sample image, code corresponding to each sample image ⌐ S1003

Train the target preset model based on the code corresponding to each sample image and the first segmentation result corresponding to each sample image, to obtain a target model ⌐ S1004

FIG. 10

FIG. 11

Input a sample image group into a target preset model — S1201

Display each sample image — S1202

Determine, in response to a mark operation performed by a user on each sample image, a seed point corresponding to each sample image — S1203

Input the seed point corresponding to each sample image into the target preset model — S1204

Perform region growing based on a seed point corresponding to a first sample image, to obtain a second segmentation result corresponding to the first sample image — S1205

Process, through a first preset neural network, the first sample image and the second segmentation result corresponding to the first sample image, to obtain a first segmentation result corresponding to the first sample image — S1206

Process, through a second preset neural network, each sample image and at least one first feature corresponding to each sample image, to obtain a feature corresponding to a segmented region in each sample image; and generate, based on the feature corresponding to the segmented region in each sample image, code corresponding to each sample image — S1207

Train the target preset model based on the code corresponding to each sample image and a first segmentation result corresponding to each sample image, to obtain a target model — S1208

FIG. 12

Seed
point

Region growing
module

Second
segmentation result

First sample
image

First preset neural
network

First segmentation
result

Label
corresponding
to the first
sample image

FIG. 13

FIG. 14

Seed point

Region growing module

Coarse segmentation result

Label corresponding to a medical sample image

Medical sample image triplet

Anchor

Positive sample

Negative sample

First preset neural network

Lesion region segmentation result

Loss function

Second preset neural network

L2 regularization

Feature corresponding to a lesion region

One-dimensional code

a  p  n

EP 4 553 675 A1

1502

First segmentation
module

1501

First input
module

1504

Retrieval
module

Encoding module

1503

FIG. 15

1602

Second
segmentation
module

1601

Second
input
module

1604

Training
module

Feature
module

1603

FIG. 16

FIG. 17

## INTERNATIONAL SEARCH REPORT

| International application No. |
|---|
| **PCT/CN2023/081658** |

| A. | CLASSIFICATION OF SUBJECT MATTER |
|---|---|

G06F16/53(2019.01)i; G06T7/00(2017.01)i

According to International Patent Classification (IPC) or to both national classification and IPC

| B. | FIELDS SEARCHED |
|---|---|

Minimum documentation searched (classification system followed by classification symbols)

G06F, G06T

Documentation searched other than minimum documentation to the extent that such documents are included in the fields searched

Electronic data base consulted during the international search (name of data base and, where practicable, search terms used)

VEN, CNTXT, USTXT, DWPI, CNKI, IEEE: 图像, 检索, 搜索, 相似, 神经网络, 分割, 感兴趣区域, 病灶, 特征, 编码, 卷积层, 融合, 尺寸, 分辨率, image, search, similar, neural network, segmentation, region of interest, ROI, lesion, feature, coding, convolution layer, fusion, disease, size, resolution

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 112559781 A (NORTHWEST UNIVERSITY) 26 March 2021 (2021-03-26) description, paragraphs [0050]-[0080] | 1-22 |
| A | CN 114612902 A (TENCENT TECHNOLOGY (SHENZHEN) CO., LTD.) 10 June 2022 (2022-06-10) entire document | 1-22 |
| A | CN 109389587 A (SHANGHAI UNITED IMAGING INTELLIGENCE CO., LTD.) 26 February 2019 (2019-02-26) entire document | 1-22 |
| A | CN 110245657 A (TSINGHUA UNIVERSITY) 17 September 2019 (2019-09-17) entire document | 1-22 |
| A | US 2020349707 A1 (HURON TECHNOLOGIES INTERNATIONAL INC.) 05 November 2020 (2020-11-05) entire document | 1-22 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 June 2023** | **12 June 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| International application No. |
|---|
| **PCT/CN2023/081658** |

| Patent document cited in search report | | | Publication date (day/month/year) | Patent family member(s) | | | Publication date (day/month/year) |
|---|---|---|---|---|---|---|---|
| CN | 112559781 | A | 26 March 2021 | None | | | |
| CN | 114612902 | A | 10 June 2022 | HK | 40070352 | A0 | 28 October 2022 |
| CN | 109389587 | A | 26 February 2019 | None | | | |
| CN | 110245657 | A | 17 September 2019 | None | | | |
| US | 2020349707 | A1 | 05 November 2020 | CA | 3138959 | A1 | 12 November 2020 |
| | | | | US | 2023014668 | A1 | 19 January 2023 |
| | | | | US | 2022230316 | A1 | 21 July 2022 |
| | | | | WO | 2020223798 | A1 | 12 November 2020 |
| | | | | EP | 3963508 | A1 | 09 March 2022 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210868965